# EUROPEAN PATENT APPLICATION

(11) **EP 2 444 071 A1**
(43) Date of publication of application: **25.04.2012**
(21) Application number: 11175018.8
(22) Date of filing: 27.09.2005
(51) Int. Cl.: A61K 9/50, A61K 31/4422, A61K 31/55

(54) **Minicapsule formulations**

(30) Priority: 27.09.2004 US 612784 P; 27.09.2004 US 612785 P; 27.09.2004 US 612786 P
(62) Divisional of application: 05786892.9
(71) Applicant: Sigmoid Pharma Limited, Dublin 9 (IE)
(72) Inventor: Moodley, Joey, County Westmeath (IE); Coulter, Ivan, Dublin 6 (IE)
(74) Representative: Harrison Goddard Foote

(57) **Abstract**

A formulation comprises a plurality of seamless minicapsules, the minicapsules having a diameter of from 0.5 mm to 5 mm, the minicapsules having a core containing an active entity and an encapsulating body, the active entity being in the form of any one or more of:-
• a microemulsion,
• a nanoemulsion,
• a self-emulsifying delivery system,
• a self-microemulsifying delivery system,
• a biostable perflurocarbon formulation,
• a complex with cyclodextrin (and the like),
• liposomes,
• hydrogel,
• lymphatic targeted delivery system,
• liquid bi-layers,
• an aqueous system,
• wax,
• emzaloid, and
• natural plant extract.

## Description

### Field of the Invention

The present invention relates to formulations utilising seamless minicapsule or minispheres, to enhance the formulation of and bioavailability of pharmaceutical and nutritional actives, including small molecules, biopharmaceuticals, veterinary actives, vaccines, immunotherapeutics, biotechnicals and nutritionals which are intended for oral, rectal, vaginal, intrauterine, nasal or pulmonary administration.

The advantages of controlled/sustained/pulsatile pharmaceutical or nutritional administration are well known. They ensure that better disease management through controlling the concentration of active that is present in the intestine or plasma at any time to that required for optimal therapeutic or nutritional benefit. Controlled release ensures both that the concentration is neither at a low sub-therapeutic nor a high toxic level. Also, pulsatile release format mimics the administration of drugs at different time points without the need for several administrations. Controlled release reduces irritation to the gastrointestinal membranes.

This invention is directed towards providing minicapsule formulations of ingredients which have heretofore proved very difficult to formulate.

### Statements of Invention

According to the invention there is provided a formulation comprising a plurality of seamless minicapsules, having a diameter of from 0.5 mm to 5 mm the minicapsules having a core containing an active entity and an encapsulating body, the active entity being in the form of any one or more of:-
a microemulsion,
a nanoemulsion,
a self-emulsifying delivery system,
a self-microemulsifying delivery system,
a biostable perflurocarbon formulation,
a complex with cyclodextrin (and the like),
liposomes,
hydrogel,
lymphatic targeted delivery system,
liquid bi-layers,
wax,
an aqueous system,
emzaloid,
a natural plant extract.

In one embodiment at least some of the minicapsules have at least one coating to control the time and/or location of the release of the active entity.

The coated seamless minicapsules may have a diameter of from 0.5 mm to 5.0 mm, from 1.2 mm to 2.0 mm, 1.4 mm to 1.8 mm.

In one embodiment at least one coating is an immediate release coating. At least one coating may be a sustained release coating. The coating may comprise a sustained release and an immediate release coating. At least one coating may be an enteric coating. At least one coating may be a bioadhesive coating. The bioadhesive coating may be a mucoadhesive coating.

In one embodiment the minicapsule comprises a buffer layer.

The minicapsule may be formed from a core solution containing an active ingredient, and an encapsulating solution which forms, on setting, the encapsulating medium. The encapsulating solution may contain an active ingredient. The active ingredient contained in the encapsulating solution may be the same as the active ingredient in the core solution. Alternatively the active ingredient contained in the encapsulating solution is different from the active ingredient in the core solution. The active ingredient contained in the encapsulating solution may be in a micronised or nanonized particle form.

In one embodiment the minicapsule is formed from a solution containing the encapsulating medium and an active ingredient.

In one case the active ingredient contained in the encapsulating solution is in a micronised or nanonized particle form.

In one embodiment the formulation comprises at least two different populations of minicapsules. One population of minicapsules may comprise minicapsules with one rate-controlling coating and another population of minicapsules comprises minicapsules with a second rate-controlling coating. One population of minicapsules may have an immediate release coating and the other population of minicapsules has a sustained or controlled release coating. One population of minicapsules may not have a coating. Alternatively or additionally one population of minicapsules contains a first active ingredient and another population of minicapsules contains a second active ingredient.

In one respect the formulation comprises a capsule containing a plurality of minicapsules. The capsule may contain another entity. The other entity may be in a liquid, powder, solid, semi-solid or gaseous form. The other entity may comprise an active entity.

In another aspect a formulation comprises a tablet or pellet containing a plurality of minicapsules. The tablet or pellet may contain another entity. The other entity may be an active entity.

In another aspect there is provided a formulation comprising a plurality of seamless minicapsules, the minicapsules having:-
(i) a core containing an active entity, the active entity being
   - solubilised in an acceptable solvent,
   - in a liquid phase,
   - in a solid form, and/or
   - in a semi-solid form
   and
(ii) an encapsulating medium (body)
   - the seamless minicapsules having a diameter of from 0.5mm to 5.0mm.

In another aspect the invention provides a formulation comprising a plurality of seamless minicapsules, the minicapsules containing an active entity in a solid and/or semi-solid form and an encapsulating medium, the seamless minicapsules having a diameter of from 0.5 mm to 5 mm. In the invention a the powder/solid material is initially dissolved/suspended in a liquid phase (e.g. gelatin). On cooling/hardening of the liquid phase, the solid material comes out of solution and is present as a solid in the hardened core. This type of formulation would generally comprise of a non-layered gelatin microcapsule core coated with an appropriate polymer. Such a formulation would be suitable for nanoparticle formulations.
- Nanoparticles (particles less than 800nm in size that are said to be taken up intact from the GIT) could be formulated as a solid core by initially dissolving the particles in gelatin before cooling (as described above).
- PEG- coated nanoparticles - solid core formulated in a similar manner to that of the nanoparticles. PEG-coated nanoparticles are capable of targeting specific tumours.

At least some of the minicapsules may have at least one coating to control the time and/or location of the release of the active entity. The coated seamless minicapsules have a diameter of from 0.5 mm to 5.0 mm, 1.2 mm to 2.0 mm, 1.4 mm to 1.8 mm.

In one embodiment at least one coating is an immediate release coating and/or a sustained release coating, and/or a sustained release and an immediate release coating, and/or an enteric coating and/or a bioadhesive coating such as a mucoadhesive coating.

In one embodiment at least some of the minicapsules comprises a buffer layer.

In one arrangement the minicapsule is formed from a core solution containing an active ingredient, and an encapsulating solution which forms, on setting, the encapsulating medium. The encapsulating solution may contain an active ingredient. The active ingredient contained in the encapsulating solution may be the same as or different from the active ingredient in the core solution. The active ingredient contained in the encapsulating solution may be in a micronised or nanonized particle form.

In another embodiment the minicapsule is formed form a solution containing the encapsulating medium and an active ingredient. The active ingredient contained in the encapsulating solution is in a micronised or nanonized particle form.

In one embodiment the formulation comprises at least two different populations of minicapsules. One population of minicapsules may comprise minicapsules with one rate-controlling coating and another population of minicapsules comprises minicapsules with a second rate-controlling coating. One population of minicapsules may have an immediate release coating and the other population of minicapsules has a sustained or controlled release coating. One population of minicapsules may not have a coating. One population of minicapsules may contain a first active ingredient and another population of minicapsules may contain a second active ingredient.

In one aspect the formulation comprises a capsule containing a plurality of minicapsules. The capsule may contain another entity. The other entity may be in a liquid, powder, solid, semi-solid or gaseous form. The other entity may comprise an active entity.

In another embodiment the formulation comprises a tablet or pellet containing a plurality of minicapsules. The tablet or pellet may contain another entity. The other entity may be an active entity.

The invention further provides a formulation comprising a plurality of seamless minicapsules, at least some of the minicapsules comprising a plurality of particles containing an active entity dispersed in an encapsulating body, the seamless minicapsules having a diameter of from 0.5 mm to 5 mm. At least some of the minicapsules have at least one coating to control the time and/or location of the release of the active entity. The coated seamless minicapsules have a diameter of from 0.5 mm to 5.0 mm, from 1.2 mm to 2.0 mm, from 1.4 mm to 1.8 mm.

At least one coating may be an immediate release coating, and/or a sustained release coating, and/or a sustained release and an immediate release coating, and/or an enteric coating, and/or a bioadhesive coating such as a mucoadhesive coating.

The minicapsule may be formed from a solution containing the encapsulating medium and an active ingredient. The active ingredient contained in the encapsulating medium may be in a micronised or nanonized particle form.

The formulation may comprise at least two different populations of minicapsules. One population of minicapsules may comprise minicapsules with one rate-controlling coating and another population of minicapsules comprises minicapsules with a second rate-controlling coating. One population of minicapsules may have an immediate release coating and the other population of minicapsules has a sustained or controlled release coating. One population may not have any rate-controlling coating. One population of minicapsules may contain a first active ingredient and another population of minicapsules may contain a second active ingredient.

The formulation may comprise a capsule containing a plurality of minicapsules. The capsule may contain another entity. The other entity may be in a liquid, powder, solid, semi-solid or gaseous form. The other entity may comprise an active entity.

The formulation may comprise a tablet or pellet containing a plurality of minicapsules. The tablet or pellet may contain another entity. The other entity may be an active entity.

The invention also provides a formulation comprising a plurality of seamless minicapsules having at least two populations selected from:-
a first minicapsule population in which the minicapsules comprise a core containing an active ingredient and an encapsulating medium, the minicapsules having a diameter of from 0.5 mm to 5 mm;
a second minicapsule population in which the minicapsules comprise a plurality of particles containing an active entity dispersed in an encapsulating medium, the minicapsules having a diameter of from 0.5 mm to 5 mm; and
a third micro or mini particles population in which the minicapsules comprise an inert core and at least one layer around the core, the layer containing an active ingredient.

In one embodiment of the various aspects of the invention at least some of the minicapsules are provided with a bioadhesive such as a mucoadhesive.

The bioadhesive may comprise from 0% to 10% by weight of one or more of the following polymer classes:- polyacrylates; polyanhydrides; chitosans; carbopols; cellulose; methylcellulose; methylated deoxycellulose (m-doc^{™}), lectins.

The bioadhesive may comprise from 0% to 10% by weight of one or more of the following thiolated or otherwise derivatised polymers:- polyacrylates; polyanhydrides; chitosans; carbopols; cellulose; methylcellulose; methylated deoxycellulose (m-doc^{™}), lectins.

The bioadhesive may comprise a coating. Alternatively or additionally the bioadhesive is incorporated into a part or layer of the minicapsule such as into the rate-controlling layer and/or into the encapsulating medium.

In another embodiment at least some of the minicapsules have a layer such as an outer layer which is divided into at least two parts. The parts may be of the same or different composition.

The minicapsules may be filled into hard gelatin capsules, or filled into a sachet, or suspended in oil as a lubricant.

The minicapsules may be contained within a wide gauge syringe that is compatible with tube delivery. The minicapsules may be in the form of a sprinkle. The minicapsules may be formulated as a suppository for rectal or vaginal or intrauterine administration, for nasal administration, and/or for oral administration of pharmaceutical, veterinary or nutritional active entities.

The minicapsules are less than 2 mm in diameter and encapsulate in the core a broad range of active formulations ranging from simple aqueous solutions to complex lipid-based matrices. Such formulations, liquid at the processing temperature and either are liquid, semi-liquid or solid at ambient temperature.

The core formulations are surrounded by a shell comprised of gelatin, starch, casein, chitosan, soy bean protein, safflower protein, alginates, gellan gum, carrageenan, xanthan gum, phtalated gelatin, succinated gelatin, cellulosephtalate-acetate, polyvinylacetate, hydroxypropyl methyl cellulose, oleoresin, polymerisates of acrylic or mthacrylic esters, polyvinylacetate-phtalate and combinations thereof, with or without an intermediate buffering layer.

The buffering layer serves to ensure that the core formulation does not interact with nor comprise the shell function. Additionally, the buffer may prevent oxidation or hydration of the core buffers.

The shell is further coated with at least one coat comprised of a bioadhesive, an enteric coat or a combination of both. Furthermore, to increase particle drug loading, the shell may have embedded in it solubilised, micronised or nanoformulated drug particles. Also, the shell may have embedded into it, various controlled release polymers and or muco- or bio-adhesives in any combination.

These minicapsules are characterised by long residence times, either in the gastric, small intestinal or large intestinal environments. Furthermore, they facilitate the delivery of one or more active entity to one or more sites of absorption or activity along the entire length of the gastrointestinal tract.

The invention provides minicapsules, comprising at least two layers, each having a core containing an active entity solubilised in an acceptable solvent or liquid phase and encapsulated into seamless multiparticulate minicapsules.

Each layer may be singly or multiply coated with at least one coating selected from the following:
at least one film forming polymer, insoluble in the digestive tract fluids, comprising at least one non-hydrosoluble cellulose derivative;
at least one nitrogen containing polymer coating, comprising at least one polyacrylate and/or poly-N-vinylamide and/or poly-N-vinyl-lactame, especially polyacrylamide and/or polyvinylpyrollidone;
at least one sulphur containing or thiolated polymer coating, comprising at least one thiolated cellulose or ethylcellulose derivative and/or a thiolated polyacrylate and/or a thiolated polyacrylamide and/or a thiolated polyvinylpyrrolidone;
at least one polymer coating, comprising of at least one cellulose, ethylcellulose or cellulose analogue, chitosan or chitosan analogues being preferred;
at least one plasticizer; and
at least one surface active and/or lubricating agent

The minicapsules may have a diameter in the range of from 0.5 to 3 mm, preferably in the range of from 1.2 to 2 mm, most preferably in the range of from 1.4 to 1.8 mm.

The minicapsules may in one case be formulated so that they are capable of extended residence times in the small intestine for a period of at least 3 hours, preferably at least 7 hours and more preferably in the 8-24 hour range to enable maximal bioactivity of the core active agent, locally or systemically.

The minicapsules may in another case be formulated so that they are capable of extended residence times in the large intestine for a period of at least 3 hours, preferably at least 7 hours and more preferably in the 8-24 hour range to enable maximal bioactivity of the core active agent, locally or systemically.

In another case the minicapsules may be formulated so that they are capable of extended residence times in the gastric environment for a period of at least 3 hours, preferably at least 7 hours and more preferably in the 8-24 hour range to enable maximal bioactivity of the core active agent, locally or systemically.

In one embodiment the shell comprises two distinct hemispherical shells. Each hemisphere may contain variable concentrations of gelatine alone or gelatine-like materials in combination with, for example, a mucoadhesive and/or an enteric material.

In one embodiment the core comprises at least one active entity in an amount of between 1-90% by weight, preferably between 5-50%.

The capsules may have an enteric coating which preferably comprises between 1.5-15% ethyl cellulose by weight.

The capsules may have a bioadhesive coating which preferably comprises between 0-10% polyvinylpyrrolidone by weight.

The bioadhesive coating may comprise between 0-10% of one or more of the following polymer classes: polyacrylates and/or chitosans and/or carbopols, and/or cellulose and the like.

Alternatively, the bioadhesive coating may comprise between 0-10% of one or more of the following thiolated or otherwise derivatised polymers: polyacrylates and/or chitosans and/or carbopols, and/or cellulose and the like.

In one embodiment the coating comprises a blend of enteric and bioadhesive agents with variable coat thicknesses.

In another embodiment the minicapsules are mixed with at least one anti-agglomerating agent, formed preferably of talc, colloidal silica or of a mixture of both.

The shell may be coated with either or both of a small intestinal-specific releasing enteric coating and/or a colon-specific releasing coating.

In this case the capsules may have an enteric undercoat comprising one or a number of mucoadhesive polymer coatings.

In one embodiment the microcapsules comprise at least two different distinct dissolution profiles.

The core active entity may comprise at least one pharmaceutical, biopharmaceutical, veterinary or nutritional entity.

The core active entity may comprise an immunostimulatory agent, for example, a vaccine or other immunotherapeutic entity, including, for example, an antigen, an adjuvant or other immunomodulator.

The core active entity may comprise live or attenuated mammalian, bacterial or viral cell culture or other formulation.

In one case the microcapsules comprise a buffer which preferably contains an active or other functional entity.

In one embodiment the shell contains an active entity.

Alternatively or additionally the core contains an active entity such as a gastrointestinal motility modulator, either in combination with other actives or separately.

The invention relates to solid oral dosage forms comprising a multiplicity of seamless minicapsules containing at least one pharmaceutically active ingredient solubilised or dispersed in a pharmaceutically acceptable solvent or liquid phase.

Coating the minicapsules with various materials as well as varying the coating thickness controls not only where along the gastrointestinal tract the minicapsule contents are release but also the residence time of the minicapsule in the small intestine. Further manipulating either the minicapsule contents or the coatings may provide additional protection of the minicapsule content from the gastric acid environment of from intestinal degradative enzyme attack. Overall, the minicapsule approach permits an increase of the effective in vivo absorption of pharmaceutical and nutritional actives.

In addition to enhancing small intestinal absorption, the present invention also permits drug release in the gastric environment or the large intestine. Furthermore, the invention enables enhanced intestinal mixing leading to increased coating of the intestinal lumen where the effect of actives in local and not systemic such as ulcerative lesions, to increase the inhibition of digestive enzymes such as lipases or to stimulate the activity of various immune cells or to manipulate the natural intestinal micro-flora or to enhance enterocirculation processes.

Finally, as the dose contained within each minicapsule is a small percent of the overall administered dose, in the 0.1-2.0 per cent range, the contents will have minimal capacity to irritate the gastrointestinal mucous membrane.

The invention provides minicapsules, comprising at least one layer, comprising at least a core containing an active entity solubilised in an acceptable solvent or liquid phase that is liquid, solid or semi-solid at room temperature and is encapsulated into seamless multiparticulate minicapsules.

The invention also provides minicapsules, comprised of at least 1 layer, the inner core comprised of an active entity solubilised or melted into a solid spherical bead forming materials such as gelatin, starch, casein, chitosan, soya bean protein, safflower protein, alginates, gellan gum, carrageenan, xanthan gum, phtalated gelatin, succinated gelatin, cellulosephtalate-acetate, polyvinylacetate, hydroxypropyl methyl cellulose, oleoresin, polymerisates of acrylic or methacrylic esters, polyvinylacetate-phtalate and combinations thereof as a solvent.

The invention further provides minicapsules, the core of which is an inert particle on which at least 1 layer is coated. Each layer may be singly or multiply coated with at least one coating selected from the following specifications:
at least one film forming polymer, insoluble in the digestive tract fluids, comprising at least one non-hydrosoluble cellulose derivative;
at least one nitrogen containing polymer coating, comprising at least one polyacrylate and/or poly-N-vinylamide and/or poly-N-vinyl-lactame, especially polyacrylamide and/or polyvinylpyrollidone;
at least one coating, comprising at least one cellulose or ethylcellulose and or chitosan or chitosan derivative;
at least one sulphur containing or thiolated polymer coating, comprising at least one thiolated cellulose or ethylcellulose derivative and/or a thiolated polyacrylate and/or a thiolated polyacrylamide and/or a thiolated polyvinylpyrrolidone;
at least one plasticizer; and at least one surface active and/or lubricating agent

The minicapsules may have a diameter in the range of from 0.5 to 5 mm, typically from 1.2 to 2 mm, or from 1.4 to 1.8 mm.

The minicapsules may be capable of extended residence times in the small intestine for a period of at least 5 hours, preferably at least 7 hours and more preferably in the 8-24 hour range to enable maximal bioactivity of the core active agent, locally or systemically.

The minicapsules may be capable of extended residence times in the large intestine for a period of at least 5 hours, preferably at least 7 hours and more preferably in the 8-24 hour range to enable maximal bioactivity of the core active agent, locally or systemically.

The minicapsules may be capable of extended residence times in the gastric environment for a period of at least 5 hours, preferably at least 7 hours and more preferably in the 8-24 hour range to enable maximal bioactivity of the core active agent, locally or systemically.

The capsule shell may comprise two hemispherical gelatine- or gelling agent-based shells. Each hemisphere may contain variable concentrations of gelatine or gelling agent-based agents alone or in combination with, for example, a mucoadhesive and/or an enteric material. Each hemisphere may contain variable concentrations of gelatine- or gelling agent-based alone or in combinations with, for example, an active pharmaceutical entity.

The core may comprise at least one active entity in an amount of between 1-90% by weight, preferably between 5-50%.

The capsules may have an enteric coating which preferably comprises between 1.5-15% ethyl cellulose by weight.

The capsules may have a bioadhesive coating which preferably comprises between 0-10% polyvinylpyrrolidone by weight.

The bioadhesive coating may comprise between 0-10% of one or more of the following polymer classes: polyacrylates, polyanhydrides, lectins and/or chitosans and/or carbopols, and/or cellulose and the like.

The bioadhesive coating may comprise between 0-10% of one or more of the following thiolated or otherwise derivatised polymers: polyacrylates, polyanhydrides, lectins and/or chitosans and/or carbopols, and/or cellulose and the like.

The coating may comprise a blend of enteric, sustained, controlled, pulsed and bioadhesive agents with variable coat thicknesses enabling controlled, sustained, pulsatile, sustained pulsed and/or controlled residence time.

The minicapsules may mixed with at least one anti-agglomerating agent, which may comprise talc, colloidal silica or of a mixture of both.

The shell may be coated with either or both of a small intestinal-specific releasing enteric coating and/or a colon-specific releasing coating.

The minicapsules may have an enteric or sustained outer layer with an undercoat comprising one or a number of mucoadhesive polymer coatings.

The capsules may comprise at least two different distinct dissolution profiles.

The core active entity may comprise at least one pharmaceutical, biopharmaceutical, veterinary, acquculture, vitamin, mineral, biotechnical, enzyme, minerals, cell, bacteria, genetically modified organism expressing pharmaceutical agent, genetically modified organism modified to survive harsh physiological environments, stem cells, adjuvant or nutritional supplement.

The core active entity may comprise an immunostimulatory agent, for example, a vaccine or other immunotherapeutic entity, including adjuvants such as Freund's complete or inosine pranobex.

The minicapsules may comprise a buffer which may contain an active or other functional entity. The shell may contain an active entity.

In one case the core contains an active entity such as a gastrointestinal motility modulator, either in combination with other actives or separately.

In one embodiment the minicapsules are encapsulating medium may be of a material selected from the group consisting of gelatin, starch, casein, chitosan, soya bean protein, safflower protein, alginates, gellan gum, carrageenan, xanthan gum, phtalated gelatin, succinated gelatin, cellulosephtalate-acetate, oleoresin, polyvinylacetate, hydroxypropyl methyl cellulose, polymerisates of acrylic or methacrylic esters, polyvinylacetate-phtalate and combinations thereof.

The minicapsules may be filled into a sachet.

The formulation may be in the form of a sprinkle, a suppository for anal or vaginal delivery, a sprinkle for buccal delivery, a sprinkle for nasal delivery, a sprinkle for pulmonary delivery, or a spinkle for intra-uterine delivery.

The formulation may contain fertility enhancers.

The formulation may contain sperm motility enhancers.

The formulation may contain donor or host eggs.

The formulation may contain sperm.

The formulation may contain sperm and sperm motility enhancers in the same or separate minicapsules.

The minicapsules of the invention are applicable to in-vitro systems, including catalysis fermentation, culturing and agriculture.

The core active entity may comprise at least one pharmaceutical, biopharmaceutical, veterinary or nutritional entity.

### Detailed Description

The invention enables enhanced controlled release of a wide range of actives in various formulations ranging from aqueous and non-aqueous liquid and semi-liquid forms as well as solid forms. The resulting form may permit any combination of single or multiple active dosage forms, any combination of formulations and any combination of controlled release profiles.

The process to produce seamless microcapsules, which provide the platform for the current invention, involves the formation of miniparticles comprised at least one layer, involves a uni-, bi- or tri-centric nozzle through which at least one material, in a liquid phase, is passed. In a one phase microsphere, the entire microsphere is manufactured from one liquid phase that solidifies at room temperature. In the two phased (layered) microcapsule, the core solution is mainly a hydrophobic solution or suspension (generally lipid based), whilst the outer shell solution generally consists of gelatin. In the three layered capsules, the core solution is hydrophilic (aqueous based) which can be encapsulated with an intermediate solution that avoids direct contact with the hydrophilic solution and the outer shell. The principle of seamless microcapsule formation is the utilization of surface tension when two different solutions contact each other, which works by reducing the contact area of the two different solutions. This requirement for the core formulation to be in a liquid phase for the manufacturing and formation of the seamless microcapsules does not however restrict the final core formulation of the minicapsules or minispheres to exclusively liquids, it is also possible to include semi - liquids (semisolids) and also solids in the core of the microcapsules. The various minicapsules or minispheres may further be processed to incorporate into or coat onto the outer shell controlled release and/or muco- or bio-adhesives, singly or in combination.

### Formulation Types

Aqueous - dissolved, micronised, nanonised, suspensions, dispersions etc Oil-/Lipid-based - emulsions, microemulsions, smedds, sedds, nanolipid emulsions, waxes, natural extracts, cells, bacterial cultures, cryo forms, other
- Drug dissolved/suspended in aqueous base (e.g. PEG 400).
- Stable liquid formulation such as glass microspheres containing antigenic material in a perflurocarbon media
- Drug dissolved/suspended in lipid base (e.g. mineral oil).
- Liposomes (microscopic, fluid-filled vesicles whose walls are made of layers of phospholipids) included in an aqueous liquid phase to entrap water soluble actives in an aqueous internal compartment. Fat-soluble medications can also be incorporated into the phospholipid layer.
- Niosomes (self assembly vesicles made from synthetic non ionic surfactants where in an aqueous solution is enclosed in highly ordered bilayer and exhibit a behaviour similar to liposomes) included in an aqueous liquid phase to entrap water soluble actives.
- Micelles (closed lipid monolayers with a fatty acid core and polar surface, or polar core with fatty acids on the surface) entrapping both hydrophobic and hydrophilic drugs in aqueous and lipid vehicles respectively.
- Polymeric micelles (Amphiphilic block copolymers such as the pluronics (polyoxyethylene polyoxypropylene block copolymers) self assemble into polymeric micelles). Hydrophobic drugs may be solubilised within the core of the micelle or alternatively, conjugated to the micelle forming polymer.
- Polymeric Vesicles (block copolymers and amphiphilic derivatives of soluble polymers such as glycol chitosan, poly-L-lysine and poly-L-ornithine that assemble into vesicular structures) self assembled in aqueous and lipid liquids allowing the incorporation of hydrophobic and hydrophilic drugs.

These vesicles can transfer materials across the cell membrane.
- Emulsions (oil and water formulations with one phase dispersed within the second phase). Emulsions may be viewed as liquid particulate systems and offer a means of administrating drugs with a low aqueous solubility (oil in water emulsions). Drugs with high aqueous solubility may also be formulated as emulsions (water in oil emulsions), and therefore potentially benefit from the association with the oil phase (lymphatic transport etc.).
- Microemulsions (isotropic blend of oils, surfactants, co-surfactants, solvents and water) can be directly incorporated into the core of the LEDDS seamless microcapsules.
- SMEDDS (self-microemulsifying drug delivery systems SMEDDS - similar to microemulsions but are formulated in the absence of water and are designed to produce a fine oil-in-water emulsion in the aqueous environment of the gut. SMEDDS can be directly formulated into the capsule core.
- SEDDS (self-emulsifying drug delivery systems) - SEDDS can also be directly formulated into the capsule core.
- Polymeric prodrugs (active substance linked via a spacer to a water soluble polymeric backbone) are solubilised in an aqueous liquid which forms the core of the seamless microcapsule.
- Lipophilic prodrugs (application of monoglyceride, diglyeride, triglyceride and phospholipid mimics for selective lymphatic transport). Lipophilic prodrugs included into the oil based core of the microcapsule.
- Lipid-coated nano- or microparticles - for enhanced permeability.
- Cyclodextrins (water-soluble cyclic carbohydrate compounds with a hydrophobic cavity due to the specific orientation of the glucosidic substituents). These compounds can be included in aqueous liquid based core formulations to form inclusion complexes with hydrophobic guest molecules (actives).
- Natural plant extracts - either natural plant extract, including sap or derivatives thereof, or extract from plants that that been genetically modified to product pharmaceutical agents, mainly, but not exclusively, biologics such as peptides, proteins and antibodies.
- Cells - encapsulation of native or genetically-modified mammalian or bacterial cells for replacement, augmentation or production of therapeutics. The cells are cultured in a solution or other form, including cryopreservatives such as glycerol, that maintains viability and activity upon release in the GIT.
- Fertility enhancers - any combination of sperm, eggs and/or fertility enhancers in a solution to aid preservation, enhance function and maximise interaction, for administration to the vagina or more specifically, the intrauterine region,
- Lipid-based emulsions to enhance permeability - formulations comprised of various combinations of medium chain unsaturated fatty acids or other permeability enhancing lipids.
- Lipid-based formulations to enhance lymphatic delivery - formulations comprised of varying length fatty acids.

### Emzaloids

Emzaloids, bi-layered vesicles comprised of essential substances which are natural and inherent components of the human body constitute a unique submicron emulsion type formulation capable of encapsulating various drugs and delivering these with high efficacy to target sites in the body. The size, shape and clustering of emzaloids can be controlled and reproduced through mechanical compositions which may enable significant advantages over other delivery systems. Emzaloids have been shown in vitro and in situ to enhance normal cell integrity and minimise cellular damage that occurs as a result of exposure to harmful effects of active ingredients.

Semi -liquid (semisolid) core formulations
- Gels - Gels (in which the active is dissolved or dispersed in a hydrophilic polymer (synthetic or natural)) may be used as the core formulation in the formation of the seamless microcapsules. Depending on the consistency and viscosity of the gel, it may be necessary for the gel to be in its liquid form prior to processing before reverting to a gel after the cooling of the microcapsules.
- Hydrogels (solid polymer matrices in which the polymer molecules are held together by covalent bonds or physical interactions). Hydrogels can offer a means of sustained drug delivery and the release kinetics from these systems may be controlled easily. Hydrogels can be formulated in the core of the LEDDS technology and may require similar processing consideration to that mentioned for the gels.
- Ointments (lotions) - can be either water or oil based and therefore can be used to formulate hydrophilic and hydrophobic drugs. Ointments are generally less viscous than gels but still might require processing deviations.
- Creams/Emulsions (creams are emulsions - they contain an oil phase, a water phase and one or more commonly, several emulgents). Cream/emulsions can also be formulated to include hydrophilic and hydrophobic drugs depending on whether the emulsion is o/w or w/o. The same manufacturing considerations apply as for the gels and ointments.
- Biliquid Foams - (stable dispersion comprising an oil-based biliquid foam and an aqueous gel). Allows for high loading of lipid soluble drugs in the oil phase which constitutes up to 90% of the formulation.
- Pastes (similar to oil based ointments but differ in that a high percentage of insoluble solid is dispersed in the base). Pastes which are generally used as protective barriers in topical formulations could be included in LEDDS formulations.
- Waxes - water- or oxygen-free formulations to enhance stability and preservation of function of labile actives and/or cells, including vaccines.

The present invention is multifaceted, comprising a core liquid/emulsion/semi-liquid or solid, one or less buffer layer, a shell with or without bioadhesive and/or gastrointestinal fluid protectants and coated with one or more bioadhesive and/or enteric coats and/or taste masking/flavoured coatings, applied singly or in combination or in interchangeable layers.

The core liquid/emulsion may contain one or more active pharmaceutical/nutritional entities that are broadly defined as water soluble, poorly water soluble or water insoluble. The core, liquid at processing temperature, may comprise aqueous or lipid-/oil-based formulations in liquid, semi-liquid or solid form. The actives may range from small molecules to biopharmaceuticals to live, attenuated or killed organisms, including mammalian, bacterial or viral cell cultures containing wild-type or genetically modified organisms. The actives may preferably be absorbed through the small intestine, be absorbed through the gastric lining, act locally at all or specific regions of the gastrointestinal tract, or be absorbed in the large intestine or activate specific cells or regions along the gastrointestinal tract, including muscosal immune functions such as M-cells and Peyer's Patches. In the case of genetically modified organisms, modified to synthesize and secret active molecules, the secreted actives may be released at sites for optimal absorption or local activity.

The buffer may contain various oils, including mineral oils. The buffering material may possess additional additives or properties, including permeability enhancement. The shell may be comprised of gelatine alone, a mixture of gelatine and/or enteric coating materials and/or bioadhesive materials and/taste masking/flavouring agents.

Additionally, the shell may contain pharmaceutical/veterinary/nutritional or stimulatory entities.

The shell may be coated with singly or combinations of enteric coating materials and/or bioadhesive materials and/or taste masking/flavouring agents as well as other functional entities, including permeability enhancement and/or other health promoting or nutritional entities.

Additionally, plasticizers, surface-active and/or lubricating agents may be added to any or all of the above minicapsule components.

To prevent problems such as caking of the coated minicapsules of the invention, at least one anti-agglomerating agent comprised of talc, colloidal silica or a combination thereof.

It is further appreciated that the present invention may be used to deliver a number of drugs, singly or in various combinations, as well as nutritional supplements or various nutritional or pharmaceutical adjuvants. The term "drug" used herein includes but is not limited to peptides or proteins (and mimetic as well as covalent, non-covalent or chemical analogue thereof), antigens, vaccines, hormones, analgesics, anti-migraine agents, anti-coagulant agents, medications directed to the treatment of diseases and conditions of the central nervous system, narcotic antagonists, immunosuppressants, immunostimulators, agents used in the treatment of AIDS, chelating agents, anti-anginal agents, chemotherapy agents, sedatives, antineoplastics, prostaglandins, antidiuretic agents, DNA or DNA/RNA molecules to support gene or other nucleic acid-based therapeutics and entities leading to various immunotherapies, including antigenic and nucleic acid-based vaccines or immunotherapies, primers and adjuvants of such as well as organisms that synthesize and secret therapeutic or health modulating entities..

Furthermore, the invention enables the development of therapies that combine a number of pharmaceutical or nutritional agents, for example, anti-infective and antimicrobial agents, such as those employed in the treatment of HIV/AIDS. In this example, multiparticulate seamless minicapsule formulation for once or twice daily administration to a patient, comprising sustained release particles each having a core containing solubilised anti-infectives in a solvent or liquid phase as a seamless minicapsule, the core being coated with at least one coat comprise a bioadhesive coat and a rate-controlling polymer coat or a coat combining both. The bioadhesive coat is comprised of substances such as carboxymethylcellulose, polyacrylates, polyanhydrides, chitosan, Carbopol® and nitrogen or sulphur/thiolated derivatives thereof as well as natural biological entities such as lectins in an amount to achieve resident times in the small intestine in the 5-24 hour range. The rate-controlling polymer coat is comprised of ammonia methacrylate copolymers in an amount sufficient to achieve therapeutically effective plasma levels of anti-infectives over at least 12 or 24 hours.

The seamless minicapsules were manufactured according to Freund Industrial Co, Ltd US Patent No 5,882,680 (Seamless Capsule and Method of Manufacturing the Same), the entire contents of which are herein incorporated by reference.

The principle of seamless minicapsule formation is the utilisation of "surface tension", when two different solutions (which are not or hardly dissolved with each other) contact each other, which works by reducing the contact area of the two different solutions.

After encapsulating the core solution which is ejected through an orifice with a certain diameter, with the shell solution which is also ejected through an outer orifice, the encapsulated sphere is then ejected into a cooling or hardening solution and the outer shell solution is gelled or solidified. This brief describes the formation of seamless minicapsules.

The core solution is mainly a hydrophobic solution or suspension.

The outer shell solution is normally gelatin based. However a hydrophilic solution can also be encapsulated with the existence of an intermediate solution, which can avoid the direct contact of the hydrophilic core solution with the outer shell.

With the nozzle having a single orifice, a minicapsule or a bead of shell/core mixed suspension can be processed resulting in a solid spherical bead-like formation.

With the nozzle having two orifices (centre and outer), a hydrophobic solution can be encapsulated.

With the nozzle having three or more orifices seamless minicapsules for various applications can be processed. (Ref US Patent No. 5,882,680). By using the above described manufacturing processing method as per US patent No. 5,882,680 for multiparticulate seamless minicapsules, anti-infective multiparticulate seamless minicapsules were produced. The completed nimodipine seamless minicapsules preferably have an average diameter of 0.50 - 3.00mm, more especially in the range 1.50 -1.80mm.

According to one embodiment a portion or all of the sustained release particles further comprise an immediate release coating applied onto the rate-controlling polymer coat, which immediate release coating comprises solubilised anti-infectives in a liquid phase.

In an alternative embodiment, the formulation can contain a portion of immediate release minicapsules each comprising a core of solubilised anti-infectives in a liquid phase.

Further formulation can contain the bioadhesive blended with the shell and coated with the rate-controlling polymer coat to ensure that the minicapsules are protected in the gastric environment but adhere to the small intestinal mucosal membrane.

Alternatively, the native minicapsule shells may alternately coated with at least one each of bioadhesive coat and an enteric coating. The alternate polymer solutions may be sprayed in a number of separate periods, between each spraying the minicapsule coatings are cured. Following the final coating, the coated minicapsules are harvested.

The formulation according to the invention may comprise at least two populations of sustained release and or bioadhesive seamless minicapsules having a least two different bioadhesive and rate-controlling profiles.

Also preferably, the formulation according to the invention provides a dissolution profile in a pre-selected media such that about 25% of the solubilised anti-infectives are released after 1 hour; 50% after 6 hours; 75% after 12 hours; 75 to 100% after 18 hours.

Also, in a preferred embodiment greater than 80% of the formulation is comprised of sustained release seamless minicapsules.

In a preferred embodiment the rate-controlling polymer coat contains Ammonia Methacrylate Copolymer Type A and Ammonia Methacrylate Copolymer Type B as described in USP/NF

Such copolymers are manufactured and marketed by Degussa GmbH, Darmstadt, Germany.

Most preferably the rate-controlling polymer coat contains a 5:95 or 10:90, 15:85 or 25:75 mixture of Eudragit RL: Eudragit RS most especially Eudragit RL 12.5:Eudragit RS 12.5 or Eudragit RL30D:Eudragit RS30D or Eudragit E100 or Eudragit E PO or a combination thereof.

Preferably the sustained release seamless minicapsules following application of the rate-controlling polymer coat are dried at a temperature of about 40-50 deg C.

In a preferred embodiment the formulation is encapsulated, for example in a hard gelatin capsule.

In another embodiment, the formulation is packaged in sachet format for sprinkle applications for mixing with soft foods or drinks.

The sustained release seamless minicapsules are formed by coating the active seamless minicapsule with the rate-controlling polymer coat comprised of ammonio methacrylate copolymers such as those sold under the Trade Mark EUDRAGIT. EUDRAGIT polymers are polymeric lacquer substances based on acrylates and/or methacrylates. The polymeric materials sold under the Trade Mark EUDRAGIT RL and EUDRAGIT RS are acrylic resins comprising copolymers of acrylic and methacrylic acid esthers with a low content of quaternary ammonium groups and are described in the "EUDRAGIT" brochure of Degussa GmbH wherein detailed physical-chemical data of these products are given. The ammonium groups are present as salts and give rise to the permeability of the lacquer films. EUDRAGIT RL is freely permeable or RS slightly permeable, independent of pH.

Minicapsules with both pH- and time-controlled release (double coating with EUDRADIT® RL/RS and EUDRAGIT® FS30D) may be prepared according to Degussa (In-vivo evaluation of EUDRAGIT™ - A novel pH- and time-controlled multiple unit colonic drug delivery system, Skalsky B., et al, Controlled Release Society 31st Annual Meeting TRANSACTIONS).

The mucoadhesive controlled GIT transit minicapsules are formed by coating the active seamless minicapsule with the transit-controlling polymer coat comprised of, for example various cellulose or cellulose derivatives such as chitosan or those sold under the brand name Carbopol®.

The rate-controlling polymer coat maybe built up by applying a plurality of coats of polymer solution or suspension to the minicapsule as hereafter described. The polymer solution or suspension contains the polymer(s) dissolved or suspended, respectively in a suitable aqueous or organic solvent or mixture of solvents, optionally in the presence of a lubricant. Suitable lubricants are talc, stearic acid, magnesium stearate and sodium stearate. A particularly preferred lubricant is talc.

The polymer solution or suspension may optionally include a plasticizing agent. Suitable plasticizing agents include polyethylene glycol, propylene glycol, glycerol, triacetin, dimethyl phthalate. diethyl phthalate, dibutyl phthalate, dibutyl sebacate or varying percentages of acetylated monoglycerides.

Suitable organic solvents include isopropyl alcohol (IPA) or acetone or a mixture.

The polymer solution or suspension maybe applied to the minicapsules preferably using an automated system such as a GLATT fluidised bed processor, Vector Flow Coater System or an Aeromatic.

Polymer solution/suspension in the quantity of 5-75 ml per kilogram of minicapsules maybe applied to the minicapsules using one of the listed automated fluidised bed processing systems to given target polymer coating weight.

In accordance with the invention the drug loaded minicapsules are coated with the rate-controlling polymers to achieve a target dissolution rate. The drug released from these minicapsules is diffusion controlled as the polymer swells and becomes permeable, it allows for the controlled release in the GIT. In order to achieve a suitable dissolution profile, the following parameters require consideration, efficient process/conditions, drug solubility/particle size, minicapsule surface area, minicapsule diameter and coating polymer suitability.

The minicapsule gelatine shell can be modified to comprise a sphere comprising two hemispheres. Each hemisphere contains variable concentrations of gelatine alone or gelatine in combination with, for example, a mucoadhesive and/or an enteric material. This aspect of the invention will ensure that the active is both in close proximity with the intestinal lumen and protected from intestinal degradative attack.

### Examples - Core Formulations

The present invention provides a controlled release formulation in solid dosage form, where the formulation comprises a multiplicity of seamless microcapsules, each of which microcapsule containing one or more active ingredients solubilised/suspended in a liquid, emulsion or semi-liquid (semisolid) phase. The components of the core formulation in which the drug is dissolved or suspended comprise any pharmaceutically acceptable solvent or liquid phase provided the solvent or liquid phase does not dissolve the wall of the microcapsules. The liquid phase often comprises an oil phase e.g. soya bean oil or mineral oil, in which the active ingredient is typically suspended. The core formulation can also comprise an aqueous phase e.g. PEG 400, in which the active ingredient is more often dissolved. When the core solution is mainly a hydrophobic solution or suspension, the microcapsules are typically bilayered with the outer shell normally being gelatin based. Hydrophilic solutions (containing dissolved or suspended active ingredient) can be encapsulated by employing an intermediate solution, which avoids direct contact of the hydrophilic core solution with the outer shell. These microcapsules are trilayered.

In addition to these conventional core formulations (lipid (oil) and aqueous phases), alternative formulations can also be encapsulated in the core of the seamless microcapsules. These alternative formulations include; emulsions, microemulsions, nanoemulsions, self emulsifying emulsions, self emulsifying microemulsions, liposome formulations, cyclodextrin formulations, biostability enhancing perfluorocarbon suspensions, liquid bilayers, hydrogels and waxes. Depending on the specific formulation, these formulations can be encapsulated in either bilayered or trilayered microcapsules as outlined previously.

The minicapsules allow the administration of pharmaceutical actives dispersed and suspended in the various formulation types (stated above) as if they are multiparticulate solid oral dosage forms. The minicapsules release their contents to the gastrointestinal tract in a manner which minimises high local concentrations of active ingredient which might otherwise result in irritation and other undesirable effects, but additionally the drug is released in an already solubilised form which aids absorption.

In one embodiment, the wall of the microcapsule is formed of soft gelatin, allowing for fast release and thus fast absorption of active ingredient (immediate release products).

Another embodiment provides an oral formulation, which allows for protection of the active ingredient from harsh environments such as gastric acid and intestinal proteases and other degradative processes. Enteric coating protects drugs from release into acidic environments, protease and nuclease inhibitors reduce proteolytic and nucleic acid degradation while mucoadhesive coatings minimise exposure to degradative enzymes. In one embodiment the enteric polymer is Eudragit S 12.5 providing zero drug release in the stomach for up to 4 hours.

In another embodiment the seamless microcapsules are coated with a rate-controlling coating to achieve therapeutically effective plasma levels of the active over at least 12 to 24 hours in a human patient. The rate-controlling coating may be of a polymeric material such as an amino methacrylate polymeric material. In one case the rate-controlling coating is an acrylate and/or methacrylate copolymer with quaternary ammonium. There may be two copolymers, one of which is highly permeable and the other, which is poorly permeable. The w/w ratio of the highly permeable polymer to poorly permeable polymer is typically from 10:90 to 15:85. In one embodiment the rate-controlling polymer coat contains Methacrylate Copolymer as a mixture of Eudragit RL:Eudragit RS. The polymeric materials sold under the trademark Eudragit RL and Eudragit RS are acrylic resins comprising copolymers of acrylic and methacrylic acid esters with a low content of quaternary groups. The ammonium groups are present as salts and give rise to the permeability of the lacquer films. Eudragit RL is freely permeable and RS is slightly permeable, independent of pH.

Eudragit polymers are polymeric lacquer substances based on acrylates and/or methacrylates. The polymer coat may be built up applying a plurality of coats of polymer solution or suspension to the minicapsule as hereafter described. The polymer solution or suspension contains the polymer(s) dissolved or suspended, respectively in a suitable aqueous or organic solvent or mixture of solvents, optionally in the presence of a lubricant. Suitable lubricants are talc, stearic acid, magnesium stearate and sodium stearate. A particularly preferred lubricant is talc. The polymer solution or suspension may optionally include a plasticizing agent. Suitable plasticizing agents include polyethylene glycol, propyleneglycol, glycerol and dibutyl sebacate. Suitable organic solvents include isopropyl alcohol (IPA) or acetone or a mixture.

### Microemulsions

### Example 1

Microemulsions are optically isotropic, transparent or translucent, low- viscous, singlephasic and thermodynamically stable liquid solutions. Microemulsions are generally formed by adding the aqueous phase, oily phase, and surfactant to a suitable vessel and mixing. If any of the ingredient is a solid, it should be added to a liquid phase in which it is soluble and heated to dissolve. For example, if the surfactant is a solid, and it is soluble in the oily phase, then it should be dissolved completely, then followed with aqueous phase, etc. On the other hand, if the surfactant is soluble in the aqueous phase, then it should first be added to the aqueous phase, dissolved completely, followed by the oily phase. Appropriate mixing devices as mentioned above can be employed for this purpose. The preparation of an oil-in-water emulsion based system, requires that the drug be dispersed into the hydrophobic material as described above, with the aqueous phase being added in the presence of surfactant or self-emulsifying hydrophobic long chain carboxylic acid ester. This procedure under suitable shear forms a microemulsion.

Cyclosporine is dissolved in a mixture of medium chain mono- and diglycerides. The surfactant Polysorbate 80 is dissolved in the aqueous phase (water). The ingredients of each phase are heated separately to 70-80°C. While mixing in an appropriate mixing device, the aqueous phase is then added to the oil phase to make 100% (w/w) mixtures. This procedure under suitable shear forms a microemulsion. The cyclosporine microemulsion is then formed into seamless microcapsules according to the methods described in US Pat. Nos. 5, 478,508 and 5,882,680 with an intermediate vegetable oil layer and an outer gelatin shell.

### Core Formulation

| Ingredients | % w/w |
|---|---|
| | |
| Cyclosporine | 10-20 |
| Medium Chain Mono and Diglycerides | 15-20 |
| Polysorbate 80 | 0-5 |
| Water | - to 100% |

### Example 1a

Sustained release cyclosporine minicapsules may also be formulated by coating the seamless minicapsules (described in Example 1), with the rate-controlling polymer coat comprised Eudragit RS and Eudragit RL. Eudragit RS and Eudragit RL are water-insoluble, swellable film formers based on neutral methacrylic acid esters with a small proportion of trimethylammonioethyl methacrylate chloride. Eudragit RL has a molar ratio of the quaternary ammonium groups to the neutral ester groups of 1:20, while Eudragit RS has a ratio of 1:40. Since quaternary ammonium groups determine the swellability and the permeability of the films in aqueous media, Eudragit RL which, contains more of these groups, form more permeable films with little delaying action. By contrast, films of Eudragit RS swell less easily and are only slightly permeable to active ingredients. Given coherent film coatings and an adequate layer thickness, it is possible to slow down drug diffusion very noticeably.

A coating solution of Eudragit RL (5% w/w) and Eudragit RS (95% w/w) dissolved in isopropyl alcohol/acetone mixture is sprayed onto the minicapsules using a GLATT fluidised bed processor. The coated minicapsules are dried in an environmentally controlled drier for between 12 to 24 hours to remove any residual solvents.

### Sustained Release Polymer Coating Solution

| Ingredients | % w/w |
|---|---|
| | |
| Eudragit RL 100 (5%) /Eudragit RS 100 (95%) | 10-15 |
| Acetone | 30-35 |
| Isopropyl alcohol | 50-60 |
| | |
| Water | 2.5-5.0 |

### Example 1b

| Core Solution | |
|---|---|
| --Stavudine USP/EP | 200 grams |
| --PEG 400 | 600 grams |
| --Tween 80 | 50-100 grams |
| --Miglycol | 50-100 grams |

| Median Solution | |
|---|---|
| --Vegetable or Mineral Oil | 1000 grams |

| Film Solution | |
|---|---|
| --Gelatin | 225 grams |
| --Sorbitol | 25 grams |
| --Purified Water | 750 grams |

| Mucoadhesive Coating Solution | |
|---|---|
| --Ethylcellulose | 5-20g |
| --PVP | 0.5-5g |
| --Castor Oil | 0-5g |
| --Magnesium Stearate | 0.5-3g |
| --Aceton | 50-300g |
| --Isopropanol | 5-50g |

| Polymer Coating Solution | |
|---|---|
| --Eudragit RL | 5% w/w |
| --Eudragit RS | 95% w/w |
| -- Minicapsule diameter | 1.50mm |

The Stavudine Multiparticulate Seamless Minicapsules were manufactured according to Freund Industrial Co. Ltd US Patent No. 5,882,680 (Seamless Capsule and Method of Manufacturing Same) and as described in the Summary of the Invention Section.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration of the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier for between 12 to 24 hours to remove any residual solvents

Encapsulation 0-100% immediate release/100-0% sustained release.

Stavudine seamless minicapsules uncoated (10% w/w by potency) and the polymer coated minicapsules (90% w/w by potency) from the above were blended using a suitable mechanical blender.

The resultant blend was filled into suitable gelatin capsules or sprinkle format to the required target strength.

### Example 1c

| Core Solution | |
|---|---|
| --Ritonavir USP/EP | 100-300 g |
| --PEG 400 | 300-600 g |
| --Polyoxyl 35 | 0-100 g |
| --Ethanol | 50-150 g |
| | |

| Median Solution | |
|---|---|
| --Vegetable or Mineral Oil | 1000 grams |

| Film Solution | |
|---|---|
| --Gelatin | 225 grams |
| --Sorbitol | 25 grams |
| --Purified Water | 750 grams |

| Mucoadhesive Coating Solution | |
|---|---|
| --Ethylcellulose | 50-200g |
| --PVP | 5-25g |
| --Castor Oil | 0-25g |
| --Magnesium Stearate | 5-50g |
| --Aceton | 50-1500g |
| --Isopropanol | 25-250g |

| Polymer Coating Solution | |
|---|---|
| --Eudragit RL | 5% w/w |
| --Eudragit RS | 95% w/w |
| -- Minicapsule diameter | 1.50mm |

The Rironavir Multiparticulate Seamless Minicapsules were manufactured according to Freund Industrial Co. Ltd US Patent No. 5,882,680 (Seamless Capsule and Method of Manufacturing Same) and as described in the Summary of the Invention Section.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration f the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (10% w/w) and Eudragit RS (90% w/w) dissolved in isopropyl alcohol/acetone mixture was sprayed onto the minicapsules using an automated fluidised bed processor. Talc was added simultaneously to avoid agglomeration.

The coated minicapsules were dried in a environmentally controlled drier for between 12 to 24 hours to remove any residual solvents

Encapsulation 5-15% immediate release/85-95% sustained release.

Ritonavir seamless minicapsules uncoated (5-15% w/w by potency) and the polymer coated minicapsules (85-95% w/w by potency) from the above were blended using a suitable mechanical blender.

The resultant blend was filled into suitable gelatin capsules to the required target strength.

### Example 1d

| Core Solution | |
|---|---|
| --Lipinavir USP/EP | 750 grams |
| -- Sorbitol | 0-125 grams |
| -- PEG | 100-400 g |
| -- Purified Water | 500-4500 g |

| Film Solution | |
|---|---|
| -- Gelatin | 225 grams |
| -- Sorbitol | 25 grams |
| -- Purified Water | 750 grams |

| Mucoadhesive Coating Solution | |
|---|---|
| --Ethylcellulose | 50-200g |
| --PVP | 5-25g |
| --Castor Oil | 0-25g |
| --Magnesium Stearate | 5-50g |
| --Aceton | 50-1500g |
| --Isopropanol | 25-250g |

| Polymer Coating Solution | |
|---|---|
| -- Eudragit RL | 5% w/w |
| -- Eudragit RS | 95% w/w |
| -- Minicapsule diameter | 1.50mm |

The Lipinavir Multiparticulate Seamless Minicapsules were manufactured according to Freund Industrial Co. Ltd US Patent No. 5,882,680 (Seamless Capsule and Method of Manufacturing Same) and as described in the Summary of the Invention Section.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration f the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (5-15% w/w) and Eudragit RS (85-95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier for between 12 to 24 hours to remove any residual solvents

### Example 1e

| Core Solution | |
|---|---|
| -- Amprenavir USP/EP | 50-350 grams |
| -- d-α, tocopheryl PEG 1000 succinate (TPGS) | 100-600 grams |
| -- PEG 400 | 50-250 grams |
| -- Propylene glycol | 10-50 grams |

| Film Solution | |
|---|---|
| -- Gelatin | 225 grams |
| -- Sorbitol | 25 grams |
| -- Purified Water | 750 grams |

| Mucoadhesive Coating Solution | |
|---|---|
| --Ethylcellulose | 50-200g |
| --PVP | 5-25g |
| --Castor Oil | 0-25g |
| --Magnesium Stearate | 5-50g |
| --Aceton | 50-1500g |
| --Isopropanol | 25-250g |

| Polymer Coating Solution | |
|---|---|
| -- Eudragit RL | 5% w/w |
| -- Eudragit RS | 95% w/w |
| -- Minicapsule diameter | 1.50mem |

The Amprenavir Multiparticulate Seamless Minicapsules were manufactured according to Freund Industrial Co. Ltd US Patent No. 5,882,680 (Seamless Capsule and Method of Manufacturing Same) and as described in the Summary of the Invention Section.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration f the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (5-50% w/w) and Eudragit RS (50-95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier for between 12 to 24 hours to remove any residual solvents

Encapsulation 5-50% immediate release/50-95% sustained release.

Amprenavir seamless minicapsules uncoated (5-50% w/w by potency) and the polymer coated minicapsules (50-95% w/w by potency) from the above were blended using a suitable mechanical blender.

### Example 2

### Core Formulation

| Ingredients | % w/w |
|---|---|
| | |
| Progesterone | 10-20 |
| Diolein/Monoolein | 15-20 |
| Cremophor EL | 0-5 |
| Water | - to 100% |

The procedure is the same as that followed in Example 1.

### SEDDS/SMEDDS/SMEOFS

### Example 3

Self-emulsifying drug delivery systems (SEDDS) are composed of natural or synthetic oils, surfactants and one or more hydrophilic solvents or co-solvents. The principal characteristic of SEDDS is their ability to form fine oil-in-water emulsions or microemulsions upon mild agitation following dilution by aqueous phases. Therapeutic agents which are included in these self-emulsifying formulations are any compound which has a biological activity and is soluble in the oil phase. The paclitaxel pre- microemulsion concentrate is then formed into seamless microcapsules according to the methods described in US Pat. Nos. 5, 478,508 and 5,882,680 with an outer gelatin shell.

A paclitaxel SEDDS formulation is prepared with polyoxyl hydrogenated castor oil. A formulation consisting of a modified vegetable oil (e.g., polyoxyl hydrogenated castor oil), a surfactant (e.g., TPGS), a co-solvent (e.g., propylene glycol) and a bile salt (e.g., sodium deoxycholate) is prepared by successive addition and mixing of each component. The paclitaxel is then added to the formulation, which is thoroughly mixed to form a clear homogenous mixture. The paclitaxel microemulsion is then formed into seamless microcapsules according to the methods described in US Pat. Nos. 5, 478,508 and 5,882,680 with an intermediate vegetable oil layer and an outer gelatin shell.

### Core Formulation

| Ingredients | % w/w |
|---|---|
| | |
| Paclitaxel | 5 |
| Unconjugated deoxycholic acid | 5 |
| Fractionated oat oil | 30 |
| Cremophor EL or TPGS | 30 |
| PEG 400 | 30 |

### Example 3a

Sustained release paclitaxel minicapsules may also be formulated by coating the seamless minicapsules (described in Example 3), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Example 4

### Core Formulation

| Ingredients | % w/w |
|---|---|
| | |
| Insulin | 5 |
| Unconjugated deoxycholic acid | 5 |
| Fractionated oat oil | 30 |
| Cremophor EL or TPGS | 30 |
| PEG 400 | 30 |

The procedure is the same as that followed in Example 3.

### Example 4a

Insulin like most biopharmaceuticals is degraded in the stomach due to the action of proteases. Gastro-protected insulin minicapsules may also be formulated by coating the seamless minicapsules (described in Example 4), with the enteric polymer, Eudragit S 12.5, providing zero drug release in the stomach up to 4 hours. Eudragit S 12.5 is plastizer free solution of Eudragit S 100 in isopropyl alcohol. Eudragit S 100 is an anionic copolymer of methacrylic acid and methacrylate. Eudragit S dissolves at pH 7.0 allowing for safe pH-triggered colonic drug delivery.

Talc and triethyl citrate are homogenised in isopropyl alcohol by means of a homogeniser for approximately 10 minutes. This suspension is poured into Eudragit S 12.5 and stirred gently during the coating process with conventional stirrers in order to avoid sedimentation. The coating solution mixture is sprayed onto the minicapsules using a GLATT fluidised bed processor. The coated minicapsules are dried in an environmentally controlled drier for between 12 to 24 hours to remove any residual solvents.

### Enteric Polymer Coating Solution

| Ingredients | % w/w |
|---|---|
| Eudragit S 12.5 | 50 |
| | |
| Triethyl citrate | 1-5 |
| Talc | 10-15 |
| Isopropyl alcohol | 40-45 |

### Example 5

### Core Formulation

| Ingredients | % w/w |
|---|---|
| Calcitonin | 5 |
| Unconjugated deoxycholic acid | 5 |
| Fractionated oat oil | 30 |
| Cremophor EL or TPGS | 30 |
| PEG 400 | 30 |

The procedure is the same as that followed in Example 3.

### Example 5a

### Core Formulation

| Ingredients | % w/w |
|---|---|
| Calcitonin (conjugated - covalent or non-covelent) | 5 |
| Unconjugated deoxycholic acid | 5 |
| Fractionated oat oil | 30 |
| Cremophor EL or TPGS | 30 |
| PEG 400 | 30 |

The procedure is the same as that followed in Example 3.

### Example 5b

Bioadhesivity (mucoadhesion) is often used as an approach to enhance the residence time of a drug in the GI tract, thereby increasing the oral bioavailability. A comparison between chitosan and other commonly used polymeric excipients indicates that the cationic polymer has higher bioadhesivity compared to other natural polymers, such as cellulose, xantham gum, and starch (Kotze et al. (1999). Bioadhesive drug delivery systems). Furthermore it has been reported that chitosan, due to its cationic nature is capable of opening tight junctions in a cell membrane. This property has led to a number of studies to investigate the use of chitosan as a permeation enhancer for hydrophilic drugs that may otherwise have poor oral bioavailability, such as peptides (Thanou et al. (2000). Intestinal absorption of octreotide: N-trimethyl chitosan chloride (TMC) ameliorates the permeability and absorption properties of the somatostatin analogue in vitro and in vivo. J Pharm Sci., 89, 951-957). Because the absorption enhancement is caused by interactions between the cell membrane and positive charges on the polymer, the phenomenon is pH and concentration dependant. Furthermore increasing the charge density on the polymer would lead to higher permeability. While chitosan provides a number of excellent properties, further derivatization of the amine functionalities can be carried out to obtain polymers with a range of properties. A number of studies demonstrated that the charge on chitosan has a role in providing intestinal permeability. Hence, a quaternary derivatised chitosan (N-trimethylene chloride chitosan) has been shown to demonstrate higher intestinal permeability than chitosan alone. The TMC derivative has used as a permeation enhancer for large molecules, such as octreotide, a cyclic peptide. Chitosan is insoluble in acidic environment so is more suited to colonic adherence which the methylated versions of chitosan such as N-trimethyl chitosan chloride (TMC) are soluble in acidic pH and is suited for adherence in the small intestine.

Bioadhesive calcitonin minicapsules are formulated by coating the seamless minicapsules (described in Example 5) with TMC. The mucoadhesive coating consists of 10-20% TMC and 1-5% magnesium stearate dissolved in an isopropyl alcohol/acetone mixture. The mucoadhesive coating solution is spray dried onto the minicapsules using an automated GLATT fluidised bed processor. The coated minicapsules are dried in an environmentally controlled drier for between 12 to 24 hours to remove any residual solvents.

In order to confer protection from the harsh environment of the stomach onto the mucoadhesive calcitonin microcapsules, the minicapsules (described above), are coated with an enteric polymer. In this instance the enteric polymer is Eudragit L 30 D-55. Eudragit L 30 D-55 is an aqueous dispersion of an anionic polymethacrylate. It is insoluble in acid media, but dissolves above pH 5.5, thereby providing zero drug release in the stomach for up to 4 hours. The combination of the Eudragit L 30 D-55 and the TMC coatings is suitable for drug delivery to the small intestine. Talc and triethyl citrate are homogenised in water using a homogeniser. The suspension is then slowly poured into the Eudragit dispersion with gentle stirring. The stirring process is continued until coating process is completed. The enteric coating solution is spray dried onto the minicapsules using an automated GLATT fluidised bed processor. The coated minicapsules are dried in an environmentally controlled drier for between 12 to 24 hours to remove any residual solvents.

### Mucoadhesive Coating Formulation

| Ingredients | % w/w |
|---|---|
| TMC | 10-20 |
| Magnesium stearate | 1-5 |
| Isopropyl alcohol/acetone | 80-90 |

### Enteric Polymer Coating Formulation

| Ingredients | % w/w |
|---|---|
| Eudragit L 30 D-55 | 50-60 |
| Talc | 5-10 |
| Triethyl citrate | 1-5 |
| Water | 35-45 |

### Example 5c

Recently the reactivity of the amine functionality on chitosan has been exploited to covalently conjugate functional excipients to the polymer backbone. Guggi and Bernkop have successfully attached an enzyme inhibitor to chitosan. The resulting polymer retained the mucoadhesivity of chitosan and further prevented drug degradation by inhibiting enzymes, such as trypsin and chymotrypsin (Guggi and Bernkop, (2003). In vitro evaluation of polymeric excipients protecting calcitonin against degradation by intestinal serine proteases. Int J Pharm. 252,187-196). This conjugated chitosan has demonstrated promise for delivery of sensitive peptide drugs, such as calcitonin.

The conjugated mucoadhesive coating solution is spray dried onto the calcitonin minicapsules (described in Example 5) using an automated GLATT fluidised bed processor. The coated minicapsules are dried in an environmentally controlled drier for between 12 to 24 hours to remove any residual solvents.

Since chitosan is insoluble in an acidic environment and is therefore more suited to colonic adherence, the minicapsules are coated with the enteric coat Eudragit FS 30 D. Eudragit FS 30 D is an aqueous 30% dispersion of an anionic polymer with methacrylic acid as a functional group. It is insoluble in acid media but dissolves by salt formation above pH 7.0. Apart from its enteric properties, its dissolution at a higher pH value allows for targeted colon delivery, as required in this instance. Talc and triethyl citrate are homogenised in water for 10 minutes before the suspension is poured into the Eudragit dispersion with gentle stirring. The spray suspension is then poured through a 0.5 mm and stirred continuously during the coating process to prevent the solids from settling. The enteric coating suspension is spray dried onto the mucoadhesive coated calcitonin minicapsules (described above) using an automated GLATT fluidised bed processor. The coated minicapsules are dried in an environmentally controlled drier for between 12 to 24 hours to remove any residual solvents.

### Mucoadhesive Coating Formulation

| Ingredients | % w/w |
|---|---|
| Conjugated Chitosan | 10-20 |
| Magnesium stearate | 1-5 |
| Isopropyl alcohol/acetone | 80-90 |

### Enteric Polymer Coating Formulation

| Ingredients | % w/w |
|---|---|
| | |
| Eudragit FS 30 D | 40-50 |
| Talc | 5-10 |
| Triethyl citrate | 0-1 |
| Water | 50-60 |

### Example 6

### Core Formulation

| Ingredients | % w/w |
|---|---|
| | |
| Paclitaxel/Docetaxel | 5 |
| Unconjugated deoxycholic acid | 5 |
| Fractionated oat oil | 30 |
| Cremophor EL or TPGS | 30 |
| PEG 400 | 30 |

The procedure is the same as that followed in Example 3.

### Example 6a

Sustained release paclitaxel/docetaxel minicapsules may also be formulated by coating the seamless minicapsules (described in Example 6), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Example 7

### Core Formulation

| Ingredients | % w/w |
|---|---|
| Progesterone | 5 |
| Unconjugated deoxycholic acid | 5 |
| Fractionated oat oil | 30 |
| Cremophor EL or TPGS | 30 |
| PEG 400 | 30 |

The procedure is the same as that followed in Example 3.

### Example 8

It is also possible to incorporate drug combinations in SEDDS/SMEDDS/SMEOFS formulations. Although many oral LEDDS formulations will provide therapeutic blood levels of the active ingredient when administered alone, it is sometimes beneficial to co-formulate a second active into the LEDDS to improve efficacy. This method is of particular use when the primary active is a substrate for P-glycoprotein. The taxanes (e.g., Paclitaxel and Docetaxel) are an example of drugs which are inhibited by P-gp and therefore will benefit from the co formulation of a bioenhancing agent for example cyclosporine (Malingré et al., (2001). Coadministration of Cyclosporine Strongly Enhances the Oral Bioavailability of Docetaxel. Journal of Clinical Oncology, 19, 1160-1166.)

### Core Formulation

| Ingredients | % w/w |
|---|---|
| Paclitaxel/Docetaxel | 2.5 |
| Cyclosporine | 2.5 |
| | |
| Unconjugated deoxycholic acid | 5 |
| Fractionated oat oil | 30 |
| Cremophor EL or TPGS | 30 |
| PEG 400 | 30 |

The procedure is the same as that followed in Example 3 with the cyclosporine being added last and dissolved quickly under mild agitation. The paclitaxel/cyclosporine pre-microemulsion concentrate is then formed into seamless microcapsules according to the methods described in US Pat. Nos. 5, 478,508 and 5,882,680 with an outer gelatin shell.

### Example 8a

Sustained release paclitaxel/cyclosporine or docetaxel/cyclosporine minicapsules may also be formulated by coating the seamless minicapsules (described in Example 8), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Biostable Perflurocarbon Formulations

### Example 9

Stability is generally conferred on very labile biomolecules (e.g. vaccines) by drying them in sugar glasses. Because of the susceptibility of these biomolecules to proteolytic attack, they must be reconstituted in aqueous media prior to i.v. administration. Once reconstituted, these vaccines require constant refrigeration to avoid losing their potency. The activity of these biomolecules can however be retained by suspension in perfluorocarbon (PFC) liquid. It is therefore possible to formulate oral vaccines (e.g., tetanus toxoid, recombinatant hepatitis B and Meningitis A conjugate) by encapsulating PFC/ glass microsphere biomolecule suspensions in a gelatin capsule (LEDDS) and subsequently coating the capsules to confer protection against proteolytic attack. It may also be possible to incorporate live or attenuated cells or organisms, including those modified to release therapeutically relevant molecules.

Glass microspheres of tetanus toxoid (prepared from drying in sugar glasses) are added to PFC liquid and agitated to obtain a homogenous suspension. The tetanus toxoid/PFC suspension is then formed into seamless microcapsules according to the methods described in US Pat. Nos. 5, 478,508 and 5,882,680 with an outer gelatin shell.

### Core Formulation

| Ingredients | % w/w |
|---|---|
| Tetanus Toxoid | 10 |
| PFC Liquid | 90 |

### Example 9a

Gastro-protected tetanus toxoid minicapsules may also be formulated by coating the seamless minicapsules (described in Example 9), with an enteric polymer. The enteric polymer may be Eudragit L 30 D-55 (providing release in the small intestine) or Eudragit FS 30 D (allowing for colonic drug targeting). Both enteric coats provide zero drug release in the stomach up to 4 hours. The formulations and coating procedures for the Eudragit L 30 D-55 and Eudragit FS 30 D are the same as that outlined in Examples 5a and 5b respectively.

### Example 10

### Core Formulation

| Ingredients | % w/w |
|---|---|
| Recombinatant hepatitis B | 10 |
| PFC Liquid | 90 |

The procedure is the same as that followed in Example 9.

### Example 10a

Gastro-protected Recombinant hepatitis B minicapsules may also be formulated by coating the seamless minicapsules (described in Example 10), with an enteric polymer. The enteric polymer may be Eudragit L 30 D-55 (providing release in the small intestine) or Eudragit FS 30 D (allowing for colonic drug targeting). Both enteric coats provide zero drug release in the stomach up to 4 hours. The formulations and coating procedures for the Eudragit L 30 D-55 and Eudragit FS 30 D are the same as that outlined in Examples 5a and 5b respectively.

### Example 11

### Core Formulation

| Ingredients | % w/w |
|---|---|
| | |
| Meningitis A conjugate | 10 |
| PFC Liquid | 90 |

The procedure is the same as that followed in Example 9.

### Example 11a

Gastro-protected Meningitis A conjugate minicapsules may also be formulated by coating the seamless minicapsules (described in Example 11), with an enteric polymer. The enteric polymer may be Eudragit L 30 D-55 (providing release in the small intestine) or Eudragit FS 30 D (allowing for colonic drug targeting). Both enteric coats provide zero drug release in the stomach up to 4 hours. The formulations and coating procedures for the Eudragit L 30 D-55 and Eudragit FS 30 D are the same as that outlined in Examples 5a and 5b respectively.

### Example 12

### Core Formulation

| Ingredients | % w/w |
|---|---|
| | |
| Probiotics (wild type) | 5-25 |
| PFC Liquid | 75-95 |

The procedure is the same as that followed in Example 9.

### Example 13

### Core Formulation

| Ingredients | % w/w |
|---|---|
| | |
| Probiotics (genetically modified) | 5-25 |
| PFC Liquid | 75-95 |

The procedure is the same as that followed in Example 9.

### Example 13a

Gastro-protected probiotic minicapsules may also be formulated by coating the seamless minicapsules (described in Example 11), with an enteric polymer. The enteric polymer may be Eudragit L 30 D-55 (providing release in the small intestine) or Eudragit FS 30 D (allowing for colonic drug targeting). Both enteric coats provide zero drug release in the stomach up to 4 hours. The formulations and coating procedures for the Eudragit L 30 D-55 and Eudragit FS 30 D are the same as that outlined in Examples 5a and 5b respectively.

### Cyclodextrins

### Example 14

Cyclodextrins (CDs) are water-soluble cyclic carbohydrate compounds with a hydrophobic cavity due to the specific orientation of the glucosidic substituients. β-cyclodextrin is most widely used in pharmaceutical applications due to cavity size, which is suitable for the widest range of drugs. CDs enhance the bioavailability of insoluble drugs by increasing the drug solubility, dissolution and/or drug permeability. CDs increase the permeability of insoluble, hydrophobic drugs by making the drug available at the surface of the biological barrier e.g., mucosa, from where it partitions into the membrane without disrupting the lipid layers of the barrier. In such cases it is important to use just enough CD to solubilise the drug in the aqueous vehicle since excess may decrease the drug availability Drugcyclodextrin complexes are prepared prior to solubilisation of the complex in an aqueous phase. The complexes can be formed by numerous physical processes including grinding, kneading, solid dispersion, solvent evaporation, co- precipitation, spray drying and freeze drying. Once the drug-CD complex has been formed, the complex is solubilised in an aqueous phase. A When added in small amounts, water-soluble polymers can enhance the CD solubilising effect by increasing the apparent complex stability constant. The polymers due to their direct participation in drug complexation, improve both pharmaceutical and biological properties of drug:CD complexes, independent of drug's physiochemical properties. Co-solvents can also improve the solubilising and stabilizing effects of CDs, for example the use of 10% propylene glycol.

A solution of docetaxel/cyclodextrin (1:1 ratio) is spray dried to produce a docetaxel/cyclodextrin complex. The docetaxel/cyclodextrin complex is then added to water and agitated. 10% propylene glycol is added to aid the solubilisation process. The docetaxel/cyclodextrin complex/water solution is then formed into seamless microcapsules according to the methods described in US Pat. Nos. 5, 478,508 and 5,882,680 with an intermediate vegetable oil layer and an outer gelatin shell.

### Core Formulation

| Ingredients | % w/w |
|---|---|
| Docetaxel/cyclodextrin complex | 30 |
| Water | 65 |
| 10% Propylene glycol | 0-5 |

### Example 14a

Sustained release docetaxel/cyclodextrin minicapsules may also be formulated by coating the seamless minicapsules (described in Example 12), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Example 15

### Core Formulation

| Ingredients | % w/w |
|---|---|
| Tolnafate/cyclodextrin complex | 30 |
| Water | 65 |
| 10% Propylene glycol | 0-5 |

The procedure is the same as that followed in Example 14.

### Example 16

Genetic-based therapies, ranging from gene replacement therapies and antisense oligonucleotides to siRNA have significant potential to treat currently untreatable diseases more efficiently and to more effectively treat other currently treatable diseases. Thus far, while much progress has been made, genetic- or nucleic acid-based therapies have encountered significant set-backs. The primary reason for failure to progress stems from poor bioavailability due a lack of effective or efficient delivery to target cells. To enhance delivery will require that the negative charge associated with nucleic acids is shielded or neutralised and/or that the thus shielded or neutralised entities are targeted to specific cells. The development of modified cyclodextrins offers much potential to overcome delivery issues. Cyclodextrin molecules modified to include cationic groups to condense and shield nucleic acids, and/or PEG-like molecules and/or modified to include an attached targeting molecule such as a homing peptide, an antibody or, for liver-specific targeting, pullulan or galactose molecules.

### Example 16a

### Core Formulation

| Ingredients | % w/w |
|---|---|
| Gene vector/amphilic cyclodextrin complex | 30 |
| Water | 65 |
| 10% Propylene glycol | 0-5 |

The procedure is the same as that followed in Example 14.

### Example 16b

### Core Formulation

| Ingredients | % w/w |
|---|---|
| siRNA/amphilic cyclodextrin complex | 30 |
| Water | 65 |
| 10% Propylene glycol | 0-5 |

The procedure is the same as that followed in Example 14.

### Example 16c

Modified cyclodextrin, including targeted and non-targeted amphiphilic cyclodextrin may be an effective drug delivery system for other polyanionic compounds incluging heparin and suramin.

### Core Formulation

| Ingredients | % w/w |
|---|---|
| herperin / amphiphilic complex | 30 |
| Water | 65 |
| 10% Propylene glycol | 0-5 |

The procedure is the same as that followed in Example 14.

### Example 16d

### Core Formulation

| Ingredients | % w/w |
|---|---|
| Suramin / amphiphilic / cyclodextrin complex | 30 |
| Water | 65 |
| 10% Propylene glycol | 0-5 |

The procedure is the same as that followed in Example 14.

### Example 16e

Other drugs that may be complexed with targeted cyclodextrin-conjugates include statins. Statins act primarily in the liver. Thus, complexing a statin with a galactoseor pullulan-conjugated cyclodextrin or cyclodextrin-derivative has the potential to significantly improve statin therapy and reduce cardiac-associated side effects.

### Core Formulation

| Ingredients | % w/w |
|---|---|
| Statin (simvastatin 10mg)/ cyclodextrin-pullulan complex | 30 |
| Water | 65 |
| 10% Propylene glycol | 0-5 |

The procedure is the same as that followed in Example 14.

### Example 16f

As in Example 16b, statins may benefit from targeting to inflammatory plaques on blood vessels, including cerebral vessels. Herein, antibody-conjugated cyclodextrins may have significant potential.

### Core Formulation

| Ingredients | % w/w |
|---|---|
| Statin (simvastatin 10mg)/ cyclodextrin-antibody complex | 30 |
| Water | 65 |
| 10% Propylene glycol | 0-5 |

### The procedure is the same as that followed in Example 14.

### Nanoemulsions

### Example 17

In contrast to microemulsions, nanoemulsions are heterogeneous systems comprised of two immiscible liquids in which one liquid is dispersed as droplets in another liquid. For the production an energy input is necessary and the obtained liquid-in liquid dispersion is thermodynamically unstable. Oil-in-water nanoemulsions present the most important nanoemulsion drug carrier systems where lipophilic drugs are dissolved in the inner phase of the emulsion. They are generally manufactured by mechanical fragmentation of an oily phase in an aqueous phase in the presence of surfactants. The very small size of the oily globules in nanoemulsions distinguishes them (advantageously) from standard emulsions in that they can convey active agents more efficiently. They also have the advantage of being able to accommodate drugs which are poorly soluble in water and simultaneously poorly soluble in the oils, by localizing the drug at the interface of o/w emulsion. In addition, nanoemulsions do not cream. The preparation of nanoemulsions requires high-pressure homogenization. The particles which are formed exhibit a liquid, lipophilic core separated from the surrounding aqueous phase by a monomolecular layer of phospholipids. The structure of such lecithin stabilized oil droplets can be compared to chylomicrons. Nanoemulsions therefore differ clearly from the liposomes, where a phospholipid bilayer separates an aqueous core from a hydrophilic external phase.

The production of nanoemuslsions involves a solvent-free high-pressure homogenization process, in which poorly soluble drugs are either incorporated into a commercial emulsion (e.g., Lipofundin and Intralipid) or alternatively a de novo production of the emulsion is performed using conventional emulsion oils (e.g., MCT and LCT oils). When starting from a preformed emulsion, either a jet-milled powder or, preferentially, a drug nanosuspension is taken and admixed to a preformed emulsion such as Lipofundin. In the de novo production, the powder or nanosuspension is admixed to a prepared coarse pre-emulsion.

Nimodipine is dissolved/suspended in a medium chain triglyceride (MCT). A coarse pre-emulsion is prepared in a rotor-stator, by adding the aqueous phase (water) to the oily phase (MCT with dissolved nimodipine), at 80° C. An emulsifying agent (e.g., lecithin) is also added. The premix is then treated five times in a high-pressure homogenizer with a pressure in the first stage of 1100 bar and a pressure in the second stage of 120 bar, with cooling to 70° C. at the outlet. The nimodipine nanoemulsion is then formed into seamless microcapsules according to the methods described in US Pat. Nos. 5, 478,508 and 5,882,680 with an intermediate vegetable oil layer and an outer gelatin shell.

### Core Formulation

| Ingredients | % w/w |
|---|---|
| Nimodipine | 10-20 |
| Lecithin | 5 |
| MCT | 25 |
| Water | - to 100% |

### Example 17a

Sustained release nimodipine minicapsules may also be formulated by coating the seamless minicapsules (described in Example 17), with the rate-controlling polymer coat comprised Eudragit RS 12.5 and Eudragit RL 12.5. The drug released from these minicapsules is diffusion controlled. As the polymer swells and becomes permeable, it allows for the controlled release in the GIT. Dibutyl sebacate, talc and magnesium stearate are mixed and finely dispersed with the diluents acetone and isopropyl alcohol. This suspension is then combined with the Eudragit polymers, Eudragit RL 12.5 (5%) and Eudragit RS (95%) and stirred gently throughout the coating process. The mixture is spray dried onto the minicapsules using a GLATT fluidised bed processor. The coated minicapsules are dried in an environmentally controlled drier for between 12 to 24 hours to remove any residual solvents.

### Sustained Release Polymer Coating Solution

| Ingredients | % w/w |
|---|---|
| Eudragit RL 12.5 (5%) /Eudragit RS 12.5 (95%) | 35-40 |
| Dibutyl sebacate | 0-0.5 |
| Talc | 1-5 |
| Magnesium stearate | 0-1 |
| Acetone | 25-30 |
| Isopropyl alcohol | 25-30 |

### Example 17b

Mucoadhesive nimodipine minicapsules may also be produced by coating the minicapsules described in Example 17 above with a suitable mucoadhesive coating. Chitosan and TMC coatings (as described in Examples 5a and 5b) can be used. Alternatively the bioadhesive coat may comprise a carboxymethylcellulose such as ethylcellulose. The bioadhesive coat should be applied in a sufficient quantity to achieve resident times in the small intestine in the 5-24 hour range.

To apply the ethylcellulose mucoadhesive coating to the nimodipine minicapsules, a coating solution of 5-15% ethylcellulose, 0-1% PVP, 0-1% castor oil and 1-2% magnesium stearate is dissolved in an isopropyl alcohol/acetone mixture and then spray dried onto the minicapsules using a GLATT automated fluidized bed processor. The coated minicapsules are dried in an environmentally controlled drier for between 12 and 24 hours to remove any residual solvents.

A sustained release coat (described in Example 17a) may be further applied to the mucoadhesive coated nimodipine seamless microcapsules.

### Mucoadhesive Coating Formulation

| | |
|---|---|
| | |

| Ingredients | % w/w |
|---|---|
| Ethylcellulose | 5-15 |
| PVP | 0-1 |
| Castor Oil | 0-1 |
| Magnesium stearate | 1-2 |
| Acetone | 70-75 |
| Isopropyl alcohol | 10-15 |

### Example 18

### Core Formulation

| Ingredients | % w/w |
|---|---|
| | |
| Cyclosporine A | 10-20 |
| Lecithin | 5 |
| MCT | 25 |
| Water | - to 100% |

The procedure is the same as that followed in Example 17.

### Example 18a

Sustained release cyclosporine A minicapsules may also be formulated by coating the seamless minicapsules (described in Example 18), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Example 19

### Core Formulation

| Ingredients | % w/w |
|---|---|
| | |
| Nimodipine | 10-20 |
| Lipofundin | - to 100% |

The procedure is the same as that followed in Example 18, except that the nimodipine is added directly to a commercially available nanoemulsion (Lipofundin) prior to homogenization.

### Example 19a

Sustained release Nimodipine minicapsules may also be formulated by coating the seamless minicapsules (described in Example 19), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 14a.

### Example 19b

Mucoadhesive nimodipine minicapsules may also be produced by coating the minicapsules described in Example 19 above with a suitable mucoadhesive coating. The formulation and coating procedure for the mucoadhesive coating is the same as that outlined in Example 17b.

A sustained release coat (described in Example 17a) may be further applied to the mucoadhesive coated nimodipine seamless microcapsules (as described in example 19a).

### Example 20

### Core Formulation

| Ingredients | % w/w |
|---|---|
| Cyclosporine A | 10-20 |
| Lipofundin | - to 100% |

The procedure is the same as that followed in Example 17, except that the cyclosporine A is added directly to a commercially available nanoemulsion (Lipofundin) prior to homogenization.

### Example 20a

Sustained release cyclosporine A minicapsules may also be formulated by coating the seamless minicapsules (described in Example 20), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Liposomes

### Example 21

Among the novel drug delivery systems, liposomes are one of the most promising, broadly applicable, and highly researched drug delivery systems. Liposomes are microscopic vesicles having single or multiple phospholipid bilayers which can entrap hydrophilic compounds within their aqueous cores. Hydrophobic compounds may partition into the phospholipid bilayers. Bilayers are usually made up of phospholipids, although other amphiphiles such as nonionic surfactants can also be employed for their construction. When phospholipids are hydrated, they spontaneously form lipid spheres enclosing the aqueous medium and the solute (drug). Liposomes can be obtained by means of several methods, i.e., use of organic solvents, mechanical procedures, removal of detergent from phospholipid/detergent mixed micelles. In general, the properties of the liposomes depend on the composition and the concentration of the constituents phospholipids, the method of phospholipid suspension, the ionic strength of the aqueous medium and the time of hydration. Drug delivery systems can also be produced by solubilisation of the candidate drug by means of complexation with cyclodextrins and further incorporation into the lipid phase of liposomes. A wide range of amphiphilic lipid are available for preparation of liposomes, but phosphatidylcholines, also known as 'lecithins', are the most commonly used in order to form the bi-layer membrane. Bilayer additive such as other phospholipids, cholesterol, fatty esters, ceramides, glycosphingolipids, tocopherol can also be included in the lipid bi-layers, in order to prevent the leakage of the encapsulated drug during storage. The stabilisation of liposomes can also be performed using polymers in between the lipid bi-layers for multi-lamelar liposomes.

Liposomes are generally formed when thin lipid films or lipid cakes are hydrated and stacks of liquid crystalline bilayers become fluid and swell. The hydrated lipid sheets detach during agitation and self-close to form a vesicle which prevents interaction of water with the hydrocarbon core of the bilayer at the edges. Once these particles have formed, reducing the size of the particle requires energy input in the form of sonic energy (sonication) or mechanical energy (extrusion).

For the purposes of these initial formulations, actives were loaded into pre-prepared empty liposomes (COATSOME® - NOF Corporation), allowing for the preparation of liposomal drugs without complicated methods (e.g., extrusion). The NOF COATSOME® is a unique research kit for liposomal drug delivery system development which removes some of the inconsistencies of encapsulating liposomal drugs.

The anti-cancer drug daunorubicin is suspended in an aqueous solution. The aqueous drug solution is then added to the freeze-dried preparation of COATSOME® at a temperature of 40°C. The drug-COATSOME® solution is then agitated, with the hydrophilic drug locating into the aqueous region of the liposome. The liposomal daunorubicin is then formed into seamless microcapsules according to the methods described in US Pat. Nos. 5, 478,508 and 5,882,680 with and an outer gelatin shell.

### Core Formulation

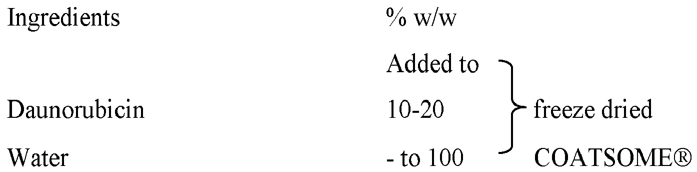

### Example 22

### Core Formulation

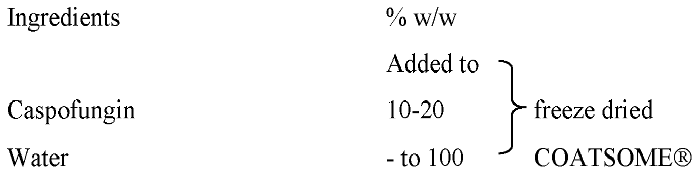

The procedure is the same as that followed in Example 21.

### Liquid Bilayers

### Example 23

Liquid bilayers comprise a dispersion of oil droplets in an aqueous medium stabilized by only a small amount of surfactant allowing for an inherent stability and also the ability to incorporate a wide range of actives. The stability is enhanced by the creation of a well-ordered self-associating bilayer or membrane. These liquid bilayers can be used to encapsulate many oliophilic liquids and additives, therefore making them very valuable for use in the pharmaceutical industry. Oils used in the liquid bilayers will in general be liquid at room temperature and may be, for example a glyceride, such as avocado oil, coconut oil, soybean oil or sunflower oil, or a caprelic/capric triglyceride, a lanolin oil, mineral oil or natural oil, or olepl alcohol, or any other oil generally known for this purpose. The oil phase can constitute up to 90% of the overall concentration of the biliquid formulation. The aqueous phase may contain water-soluble or water-dispersible materials commonly used pharmaceutical formulations, such as an alcohol (e.g., ethanol or propanol), a glycol (e.g., propylene glycol), glycerin, or any other water-soluble material generally known for this purpose. The formulation may contain, as described above, a low level of a surfactant which may be, for example an amidoamine, an acyl-lactate, an ester of a polyhydric alcohol (e.g., an ester of an ethylene, diethylene or propylene glycol), a polyoxyethylene or polyoxypropylene derivative of an alcohol, amide or ester, an alkyl ether sulphate, an alkyl betaine or a suitable compatible mixture of these surfactants.

Cyclosporine A is dissolved in mineral oil. The primary surfactant (polyoxyethylene lauryl ether) is then added to the oil phase. This oil is then mixed with the aqueous phase phase, 5% by weight of a coactive surfactant (lauryl betaine). The cyclosporine A biliquid is then formed into seamless microcapsules according to the methods described in US Pat. Nos. 5, 478,508 and 5,882,680 with and an outer gelatin shell.

### Core Formulation

| Ingredients | % w/w |
|---|---|
| Cyclosporine A | 10-20 |
| Mineral oil | 80-90 |
| Water | 10-20 |
| Lauryl betaine | 0.05-0.1 |
| Polyoxyethylene lauryl ether | 1.0 -2.0 |

### Example 23a

Sustained release cyclosporine A minicapsules may also be formulated by coating the seamless minicapsules (described in Example 23), with the rate-controlling polymer coat comprised Eudragit RS and Eudragit RL. The formulation and coating procedure for the Eudragit RL (5% w/w) and Eudragit RS (95% w/w) coating solution is the same as that outlined in Example 1a.

### Hydrogels

### Example 24

Hydrogels are solid polymer matrices in which the polymer molecules are held together by covalent bonds or physical interactions. These usually offer a means of sustained drug delivery.

Insulin bioadhesive hydrogels are prepared from acrylic block copolymers of methacrylic acid and methacrylate. Sodium salts of various fatty acids were incorporated into the hydrogels as absorption enhancers. The insulin hydrogel is then formed into seamless microcapsules according to the methods described in US Pat. Nos. 5, 478,508 and 5,882,680 with an intermediate oil layer and an outer gelatin shell.

### Core Formulation

| Ingredients | % w/w |
|---|---|
| Insulin | 10-20 |
| Methacrylic acid | 20-40 |
| methacrylate. | 20-40 |
| Sodium lauric acid | 1-5 |
| Water | - to 100 |

### Example 24a

Gastro-protected insulin minicapsules may also be formulated by coating the seamless minicapsules (described in Example 24), with the enteric polymer, Eudragit S 12.5, providing zero drug release in the stomach up to 4 hours. The Eudragit S 12.5 protective coat is formulated and applied as described in example 4a.

### Polymer Coating Solution

| Ingredients | % w/w |
|---|---|
| Eudragit S 12.5 | 50 |
| Triethyl citrate | 1-5 |
| Talc | 10-15 |
| Isopropyl alcohol | 40-45 |

### Lymphatic targeted delivery systems

### Example 25

Since oral administration is relatively safe and is convenient for the patient, much of the focus in relation to lymphatic delivery has concentrated on this route. Since the rate of lymph flow in the intestine is approximately 500 times less than that of the blood flow in the portal vein, unless selective uptake into the lymphatics occurs, most drugs will be transported almost exclusively by the portal circulation. It is however known that certain highly lipophilic compounds with significant solubilites in a triglyceride lipid are transported via the lymphatic route. In addition, the transport of these compounds may be enhanced by co-administration of specific types of lipid vehicle.

The prodrug approach has been examined in an attempt to achieve significant lymphatic transport following oral administration. The aim is to synthesis promoieties with enhanced lipophilicity, which are metabolically and chemically stable during absorption and transport phases of delivery to the lymph, and, which undergo conversion selectively at sites other than the liver.

Epitiostanol (EP) is a lipophilic epithiosteroid with anti-estrogenic activity making it a useful therapy for the treatment of breast cancer. It is only clinically effective when administered by intramuscular injection as extensive first pass metabolism occurring in the intestinal mucosa and the liver precludes its oral administration (Xenobiotica, 1991, 21, 865-872). Mepitiostane (MP) is the 17-substituted methoxycyclopentane derivative of EP, and has been designed as a prodrug for avoiding the extensive first pass metabolism associated with oral administration of EP. The clinical profile of orally administered MP has been shown to be similar to intramuscularly administrated EP in terms its anti-estrogenic activity. Studies have shown that MP (orally active form of EP) avoided the first pass metabolism as a result of its selective lymphatic absorption (Xenobiotica, 1991, 21, 873-880).

Using the LEDDS technology the oral delivery of mepitiostane to the lymphatic system can be further enhanced by dissolving/suspending the prodrug in a lipid vehicle which acts to enhance transport of the lipophilic compound to the lymphatics.

Mepitiostane is solubilised/suspended in linseed and formed into seamless microcapsules with an outer gelatin coating according to the methods described in US Pat. Nos. 5, 478,508 and 5,882,680. The core and shell formulations are outlined below.

### Core Formulation

| Ingredients | % w/w |
|---|---|
| Mepitiostane | 10-20 |
| Linseed oil | 80-90 |

### Shell Solution

| Ingredients | % w/w |
|---|---|
| | |
| Gelatin | 15-20 |
| Sorbitol/Glycerin | 1-5 |
| Purified Water | 70-80 |

The mepitiostane minicapsules can be gastro-protected by coating the seamless minicapsules (described above), with the enteric polymer, Eudragit S 12.5, providing zero drug release in the stomach up to 4 hours. The Eudragit S 12.5 5 coating solution and coating procedure is the same as that outlined in Example 4a.

### Example 25b - Small Intestine

A combination of a Eudragit L 30 D-55 coat and a mucoadhesive TMC coat (suitable for drug delivery to the small intestine) may be applied to the mepitiostane minicapsules. The formulation and coating procedure for the Eudragit L 30 D-55 and mucoadhesive TMC coats are the same as that outlined in Example 5b above.

### Example 25c - Large Intestine

In another embodiment, a combination of a Eudragit FS 30 D coat and a mucoadhesive chitosan coat (suitable for colonic delivery) may be applied to the mepitiostane minicapsules. The formulation and coating procedure for the Eudragit FS 30 D and mucoadhesive chitosan coats are the same as that outlined in Example 5b above.

### Example 26

Other approaches to lymphatic transport include the targeting of macromolecules to the lymphatic system. As a result of the size of these macromolecules, they are poorly absorbed into the blood system and therefore are targeted towards the relatively open structure of the lymphatic endothelium. Lymphotropic delivery systems, where the drug is complexed with a high molecular weight carrier e.g. dextran sulphate, are used in the presence of absorption enhancers to potentiate the selective lymphatic delivery of anti-cancer drugs.

Using the LEDDS technology the oral delivery of vinblastine complexed with dextran sulphate to the lymphatic system can be enhanced by dissolving/suspending the anti-cancer agent complex in a lipid vehicle which acts to enhance transport of the lipophilic compound to the lymphatics.

The vinblastine/dextran sulphate complex is solubilised/suspended in linseed and formed into seamless microcapsules with an outer gelatin coating according to the methods described in US Pat. Nos. 5, 478,508 and 5,882,680. The core and shell formulations are outlined below.

### Core Formulation

| Ingredients | % w/w |
|---|---|
| | |
| Vinblastine/dextran sulphate complex | 10-20 |
| Linseed oil | 80-90 |

### Shell Solution

| Ingredients | % w/w |
|---|---|
| | |
| Gelatin | 15-20 |
| Sorbitol/Glycerin | 1-5 |
| Purified Water | 70-80 |

The vinblastine/dextran sulphate minicapsules can be gastro-protected by coating the seamless minicapsules (described above), with the enteric polymer, Eudragit S 12.5, providing zero drug release in the stomach up to 4 hours. The Eudragit S 12.5 5 coating solution and coating procedure is the same as that outlined in Example 4a.

### Example 26a

A combination of a Eudragit L 30 D-55 coat and a mucoadhesive TMC coat (suitable for drug delivery to the small intestine) may be applied to vinblastine/dextran sulphate minicapsules. The formulation and coating procedure for the Eudragit L 30 D-55 and mucoadhesive TMC coats are the same as that outlined in Example 5b above.

### Example 26b

In another embodiment, a combination of a Eudragit FS 30 D coat and a mucoadhesive chitosan coat (suitable for colonic delivery) may be applied to the vinblastine/dextran sulphate minicapsules. The formulation and coating procedure for the Eudragit FS 30 D and mucoadhesive chitosan coats are the same as that outlined in Example5b above.

### Encapsulated Enzymes

### Example 27

Artificial cells containing adsorbents have long been used routinely in hemoperfusion to treat acute poisoning, high blood aluminum and iron, and as a supplement to dialysis in kidney failure.

A physiological process, enterocirculation, facilitates the removal of toxic substances from the bloodstream. This is enabled by the creation of a gradient across the intestinal wall. Enterocirculation has been harnessed through delivery of artificial cells that contain various enzymes to treat hereditary enzyme deficiency diseases and other diseases. Following oral delivery of specific amino acids, enterorecirculation of such amino acids in the intestine led to the depletion of specific amino acids in the bloodstream (Artificial cells with emphasis on bioencapsulation in biotechnology; Chang TM; Biotechnol Annu Rev. 1995; 1:267-95). Clinical studies have demonstrated that artificial cells microencapsulated asparaginase, glutaminase and tyrosinase given orally can deplete the corresponding amino acid from the intestine.

More recently the Chang group lowered systemic tyrosine in animal studies and found that two intravenous injections of polyhemoglobin-tyrosinase followed by three times a day oral administration of encapsulated tyrosinase could immediately lower the body tyrosine and maintain this low level as long as the oral administration is continued (Effects of Combined Oral Administration and Intravenous Injection on Maintaining Decreased Systemic Tyrosine Levels in Rats, Yu and Chang; Artificial Cells, Blood Substitutes, and Biotechnology (formerly known as Artificial Cells, Blood Substitutes, and Immobilization Biotechnology, Volume 32, Number 1 (2004), Pgs 129 - 148)

Phenylketonuria (PKU) is an inborn error of amino acid metabolism caused by phenylalanine hydroxylase (PAH) deficiency. Dietary treatment has been the cornerstone for controlling systemic phenylalanine (Phe) levels in PKU for the past 4 decades. Over the years, it has become clear that blood Phe concentration needs to be controlled for the life of the patient, a difficult task taking into consideration that the diet becomes very difficult to maintain. Therefore alternative models of therapy are being pursued. There is an increasing interest in enzyme replacement therapy (ERT) for metabolic diseases. Two enzyme systems are being developed for treatment of PKU: the PAH enzyme and the Phe-degrading enzyme from plants; phenylalanine ammonia-lyase (PAL). PAL therapy for PKU has some advantages. PAL requires no cofactors for degrading Phe, and its by-product trans-cinnamate has a very low toxicity and no embryotoxic effects in experimental animals. Trans-cinnamic acid is converted in the liver to benzoic acid, which is then excreted via the urine mainly as hippurate. A non-mammalian enzyme, PAL is widely distributed in plants and some fungi and yeasts and also produced from E. coli. PAL was investigated to treat PKU as early as 1980 and ERT studies in human PKU patients began with the oral administration of PAL in enteric-coated gelatin capsules. The purified PAL from the yeast *R. glutinis* was packed into hard gelatin and enteric-coated capsules. PAL enteric-coated capsules reduced the blood Phe levels in PKU patients by 22% (Lancet, 1980. Enzymatic control of phenylalanine intake in phenylketonuria. 23, 392 - 394).

The oral delivery of PAL poses a number of difficulties. PAL is degraded by the acidic conditions of the stomach and therefore must be protected. PAL from *Rhodosporidium toruloides* was reported to have no activity at pH 2.2 and a half-life in duodenal juice of 3.5 minutes. PAL from R. glutinis was also inactivated rapidly by duodenal juice. This inactivation of PAL in duodenal juice was due to the enzyme being more susceptible to chymotrypsin than to trypsin. (Biochem. Biophys. Res. Commun., 1985. 131, 557 - 563).

PAL is poorly soluble and therefore ideally should be presented in a multiparticulate format to maximize the dissolution rate. Therefore formulation as a conventoional tablet is not suitable. The enteric coating of hard gelatin capsules described above is a difficult process resulting in unsightly capsules which are generally large and can prove difficult to swallow.

The current invention permits amino acid, peptides or proteins as well cross-linked or other derivatives thereof to be encapsulated in minicapsules and thereafter coated both with an enteric coat to protect the minicapsule from dissolution in the harsh gastric acidic environment and a mucoadhesive coat to ensure that the encapsulated amino acids, peptides, proteins or derivatives thereof are released at the intestinal lining, thus ensuring that the intestinal-bloodstream concentration gradient facilitates maximum and rapid enterocirculation. The pH optima of PAL (produced from R. *glutinis and Rhodotorula rubra*) are 8.75 and 8.0 respectively. Therefore it is essential that PAL encapsulated in LEDDS be targeted for colonic delivery (in order for PAL to be made available near its optimal and pH and also to avoid degradation in the upper regions of the GIT).

PAL is solubilised/suspended in a suitable medium chain triglyceride (MCT) and formed into seamless microcapsules with an outer gelatin coating according to the methods described in US Pat. Nos. 5, 478,508 and 5,882,680. The core formulation is outlined below. The shell formulation is the same as that outlined in Example 25. The encapsulated PAL may be crosslinked or non-crosslinked.

### Core Formulation

| Ingredients | % w/w |
|---|---|
| | |
| PAL (crosslinked or non-crosslinked) | 10-20 |
| MCT | 80-90 |

As mentioned above, PAL is susceptible to chymotrypsin. As outlined in Example 5b, the reactivity of the amine functionality on chitosan has been recently exploited to covalently conjugate functional excipients to the polymer backbone. These functional excipients include enzyme inhibitors for enzymes including trypsin and chymotrypsin.

The conjugated mucoadhesive coating solution is spray dried onto the PAL minicapsules (described above) using an automated GLATT fluidised bed processor. Since chitosan is insoluble in an acidic environment and is therefore more suited to colonic adherence, the minicapsules are coated with the enteric coat Eudragit FS 30 D. The colonic targeted Eudragit FS 30 D coat allows protection from the gastric conditions of the stomach and the also the lower pH of the duodenum. The formulations and coating procedures for both the mucoadhesive and enteric coats are the same as that outlined in Example 5b. The enteric coating suspension is spray dried onto the mucoadhesive coated PAL minicapsules (described above) using an automated GLATT fluidised bed processor.

### Example 28

There is at present no effective treatment for melanoma, a fatal skin cancer, which now represents the fifth most common type of cancer in North America. One unique characteristic of melanoma cells is that they need higher concentration of tyrosine for growth than that for normal cells. L-Tyrosine is an amino acid derived from protein degradation, dietary intake, and phenylalanine hydroxylation. Systemic tyrosine level can be reduced by the use of a low tyrosine diet. However, it takes a long time for this diet to lower systemic tyrosine. Furthermore, this diet is not well tolerated, resulting in nausea, vomiting, and weight loss. The injection of the enzyme, tyrosinase, which catalyzes the conversion of L-tyrosine into L-dopa-quinone, by itself, is also not practical because the short half-life of a few minutes after intravenous injection and thus requiring repeated injection resulting in immunological problems.

Oral administration of tyrosinase poses similar difficulties to that outlined for PAL in Example 26 above (i.e. gastric degradation, proteolytic attack, and poor solubility). In addition to allowing these difficulties to be overcome, the LEDDS technology has potential advantages in enzyme therapy for the lowering of systemic tyrosine when compared with earlier approaches in experimental enzyme therapy, based on parenteral injections or extracorporeal circulation of blood. These include the absence of parental or surgical intervention and the improvement in enzyme stability. Tyrosinase can also be used as a cancer vaccine. Unlike conventional vaccines that are used to prevent infectious disease, cancer vaccines attempt to stimulate the body's own immune system to recognize cancer cells as foreign and destroy them. Tyrosinase, a normal protein present in melanin (which gives moles their color), and in melanoma cells has been administered to patients to help helps "energize" their immune system, stimulating the patients immune T-cells to recognize the tyrosinase. It is proposed that these same T-cells would recognize the patients' melanoma cells (which also contain the tyrosinase) and destroy them.

Tyrosinase is solubilised/suspended in a suitable medium chain triglyceride (MCT) and formed into seamless microcapsules with an outer gelatin coating according to the methods described in US Pat. Nos. 5, 478,508 and 5,882,680. The core formulation is outlined below. The shell formulation is the same as that outlined in Example 25.

The encapsulated tyrosinase may be crosslinked or non-crosslinked.

### Core Formulation

| Ingredients | % w/w |
|---|---|
| | |
| Tyrosinase (crosslinked or non-crosslinked) | 10-20 |
| MCT | 80-90 |

Similarly to PAL, tyrosinase is susceptible to intestinal proteolytic attack. The tyrosinase LEDDS capsules (described above) are therefore coated with chitosan conjugated with an enzyme inhibitor. The formulation and coating procedure for the conjugated mucoadhesive coating solution is the same as that outlined in Example 5b.

The activity of tyrosinase is higher at a pH above pH 6. Therefore the tyrosinase mucoadhesive minicapsules are colonic targeted by coating with a Eudragit FS 30 D coat. The formulation and coating procedure for both the Eudragit FS 30 D coat is the same as that outlined in Example 5b.

### Capsule-minicapsule Cavity

When minicapsules are inserted into hard gelatine capsules, depending on the minicapsule size, a vacuum or interstitial or inter-minicapsule space exists. This space may be filled with various liquids, semi-liquids, powders or gases containing various active or inert entities, including drugs, excipients and preservatives. The filling material may be blended with minicapsules prior to filling hard gelatine capsules with the blended liquid, powder or gas.

### Pill Format

Apart from insertion into hard gelatine capsules, minicapsules also may be blended with various excipients and/or actives prior to being pressed into tablet, pellet or pill formats that may further be coated with various controlled release polymers. Additionally, such pill formats may erode over time permitting controlled release of the minicapsules.

In a further embodiment, the tablet, pellet or pill format may be gastric retentive and swell in the stomach, preventing passage into the small intestine, thus releasing the minicapsule contents at variable rates within the stomach.

In all cases of the seamless microcapsules technology an active entity may be dispersed in an encapsulating medium, the same or a different active entity may be present in a core, and/or the same or a different active entity may be present in a layer or coating. The active entity may be in a solid or semi-solid form. An active entity solubilised in a solvent or in a liquid phase may alternatively or additionally be present in a core.

There may be a number of different proportions containing the same or different actives in the formulation. Such populations in turn may have sub-populations, for example an active formulation for immediate release, controlled or sustained release with various coatings/layers to control the time and/or location of release of the active. A wide range of possiblities exist within the scope of the invention.

For example, a formulation may comprise a plurality of seamless microcapsules having at least two populations selected from:-
a first minicapsule population in which the minicapsules comprise a core containing an active ingredient and an encapsulating medium, the minicapsules having a diameter of from 0.5 mm to 5 mm;
a second minicapsule population in which the minicapsules comprise a plurality of particles containing an active entity dispersed in an encapsulating medium, the minicapsules having a diameter of from 0.5 mm to 5 mm; and
a third micro or mini particles population in which the minicapsules comprise an inert core and at least one layer around the core, the layer containing an active ingredient.

An example of an active pharmaceutical ingredient used as a model to demonstrate the range of formulations possible is nimodipine. Formulations of a dihydropyrimidine are described in our co-pending PCT application entitled "Dihydropyrimidine Formulations" filed September 27, 2005, the entire contents of which are herein incorporated by reference. Nimodipine is a dihydropyridine derivative and belongs to the class of pharmacological agents known as calcium channel blockers. The contractile processes of smooth muscle cells are dependent upon calcium ions, which enter these cells during depolarisation as slow ionic transmembrane currents. Nimodipine inhibits calcium ion transfer into these cells and thus inhibits contractions of vascular smooth muscle. Nimodipine is a yellow crystalline substance, practically insoluble in water. Nimodipine is typically formulated as soft gelatine capsules for oral administration. Nimodipine is indicated for the improvement of neurological outcome by reducing the incidence and severity of ischemic deficits in patients with subarachnoid haemorrhage from ruptured intracranial berry aneurysms regardless of their post-ictus neurological condition. The precise mode of action is not clear.

### Example 29

| Core Solution | |
|---|---|
| --Micronised Nimodipine USP/EP | 11.7% |
| --PEG 400 | 46.6% |

| Median Solution | |
|---|---|
| -- Medium-Chain Triglycerides (MCT) | 2.4% |
| -- Sucrose Acetate Isobutylate (SAIB) | 9.4% |

| Film Solution | |
|---|---|
| -- Gelatin | 30% |
| -- Purified Water | as required |
| | |
| -- Minicapsule diameter | 1.50-1.80 mm |

The Immediate Release (IR) Nimodipine Multiparticulate Seamless Minicapsules were manufactured according to Freund Industrial Co. Ltd US Patent No. 5,882,680 (Seamless Capsule and Method of Manufacturing Same) and as described in the Summary of the Invention Section. The multiparticulate minicapsules produced in this example achieved an Immediate Release Dissolution Profile as follows.

### Dissolution Method

| | |
|---|---|
| Apparatus: | Vankel VK7025 fully auto mated with Cary Win UV |
| Dissolution Medium: | Gastric Juice with 1% SDS pH 1.2 (900mls) |
| Stirring: | USP Apparatus 2 (Paddles) at 100rpm |
| UV: | 330nm |

Dissolution Profile of Nimodipine Multiparticulate Immediate Release Seamless Minicapsules Batch MY11

| | | | | | | | | | | | | | | | | | |
|---|---|---|---|---|---|---|---|---|---|---|---|---|---|---|---|---|---|
| Time (Mins) | 0 | 1 | 3 | 4 | 6 | 7 | 9 | 10 | 12 | 13 | 15 | 16 | 18 | 19 | 21 | 22 | 24 |
| | | 5 | 0 | 5 | 0 | 5 | 0 | 5 | 0 | 5 | 0 | 5 | 0 | 5 | 0 | 5 | 0 |
| Batch MY11 | 0 | 8 | 2 | 3 | 4 | 5 | 5 | 64 | 69 | 74 | 78 | 82 | 85 | 89 | 92 | 95 | 99 |
| % Released | | | 2 | 4 | 4 | 2 | 8 | | | | | | | | | | |

The immediate release product was then filled into hard gelatine capsules to the required dosage strength. Furthermore the invention allows for the immediate release product to be produced in combination with a Sustained Release or Controlled Release multiparticulate minicapsule product in varying ratio's of IR:SR/CR. The immediate release minicapsules can be combined with a Sustained or Controlled release minicapsule component in the following ratio's (w/w by potency) eg. 10% Immediate Release (IR)+90% Sustained (SR)/Controlled Release (CR) minicapsules; 20% IR + 80% SR/CR; 30% IR + 70% SR/CR; 40% IR + 60% SR/CR and 50% IR + 50% SR/CR.

### Example 30

| Core Solution | |
|---|---|
| -- Micronised Nimodipine USP/EP | 11.7% |
| -- PEG 400 | 46.6% |

| Median Solution | |
|---|---|
| -- MCT | 2.4% |
| -- SAIB | 9.4% |

| Film Solution | |
|---|---|
| -- Gelatin | 20.2% |
| -- Sorbitol | 3.0% |
| -- Hydroxypropylmethyl Cellulose Phthlate (HP55) | 6.1% |
| -- Sodium Hydroxide (NaOH) | 0.7% |

The above Example 30 were manufactured according to Freund Industrial Co. Ltd US Patent No.5,882,680 (Seamless Capsule and Method of Manufacturing Same).

In order to control the release (SR) of the Nimodipine over an extended period of time, Hydroxypropylmethyl Cellose Phthalate (HP55) was added to the Film Solution to act as a retarding agent which controlled the release of the Nimodipine over a given period. The multiparticulate minicapsules produced in this example achieved a Sustained/Controlled Release Dissolution Profile as follows.

### Dissolution Method

| | |
|---|---|
| Apparatus: | Vankel VK7025 fully auto mated with Cary Win UV |
| Dissolution Medium: | Gastric Juice with 1% SDS pH 1.2 (900mls) |
| Stirring: | USP Apparatus 2 (Paddles) at 100rpm |
| UV: | 330nm |

Dissolution Profile of Nimodipine Multiparticulate Sustained Release Seamless Minicapsules Batch MY21 1

| | | | | | | | | | | | | | | | | | |
|---|---|---|---|---|---|---|---|---|---|---|---|---|---|---|---|---|---|
| Time (Mins) | 0 | 1 | 3 | 4 | 6 | 7 | 9 | 10 | 12 | 13 | 15 | 16 | 18 | 19 | 21 | 22 | 24 |
| | | 5 | 0 | 5 | 0 | 5 | 0 | 5 | 0 | 5 | 0 | 5 | 0 | 5 | 0 | 5 | 0 |
| Batch MY21 | 0 | 2 | 3 | 3 | 4 | 6 | 1 | 21 | 32 | 44 | 55 | 65 | 74 | 82 | 89 | 96 | 10 |
| % Released | | | | | | | 1 | | | | | | | | | | 1 |

The resultant multiparticulate minicapsules were filled into suitable hard gelatin capsules to the required target strength, typically 30/60/90/120 or 180 mg Furthermore the invention allows for the combination of the SR/CR multiparticulate minicapsule with an immediate release multiparticulate minicapsule in varying ratio's of SR/CR: IR (%percent Example 30+29). The IR + SR/CR combination ratio's are as per Example 29.

### Example 31

| Core Solution | |
|---|---|
| -- Micronised Nimodipine USP/EP | 37.5% |
| -- Gelatin | 56.3% |
| -- Sorbitol | 6.3% |
| -- Purified Water | as required |

| Polymer Coating solution | |
|---|---|
| -- Eudragit RS | 85% w/w |
| -- Eudragit RL | 5% w/w |
| -- Dibutyl Sebacate | 10% w/w |
| -- Talc | as required |
| -- Minicapsule Diameter | 1.50 1.80mm |

The above seamless minicapsules were manufactured in the same way as Example 29 & 30 with the following exceptions: -
1. The core solution was treated with a High Pressure Homogeniser.
2. The median and film solutions were excluded from this example.
3. The polymer coating solution included a 10% plasticiser. The Eudragit RS/RL were adjusted proportionately.

The process used to manufacture the multiparticulate minicapsules in this example in principle was the same as used in Example 29 & 30 with the exception that only a single orifice dosing system was used instead of the normal multiple dosing orifice system. By using a single dosing orifice a uniform solid gelatine pellet or sphere is produced to a specified particle size. This method produces a durable sphere in a gelatine format that includes the active ingredient which in turn allows the sphere or multipaticulate pellet to be further processes with various polymer coating systems. The multiparticulate minicapsules produced in this example achieved a Sustained/Controlled Release Dissolution Profile as follows.

### Dissolution Method

| | |
|---|---|
| Apparatus: | Vankel VK7025 fully auto mated with Cary Win UV |
| Dissolution Medium: | Gastric Juice with 1% SDS pH 1.2 (900mls) |
| Stirring: | USP Apparatus 2 (Paddles) at 100rpm |
| UV: | 330nm |

Dissolution Profile of Nimodipine Multiparticulate Sustained Release Seamless Minicapsules Batch MY22

| | | | | | | | | | | | | | | | | | |
|---|---|---|---|---|---|---|---|---|---|---|---|---|---|---|---|---|---|
| Time (Mins) | 0 | 1 | 3 | 4 | 6 | 7 | 9 | 10 | 12 | 13 | 15 | 16 | 18 | 19 | 21 | 22 | 24 |
| | | 5 | 0 | 5 | 0 | 5 | 0 | 5 | 0 | 5 | 0 | 5 | 0 | 5 | 0 | 5 | 0 |
| Batch MY22 | 0 | 2 | 3 | 3 | 4 | 5 | 6 | 8 | 10 | 12 | 15 | 19 | 22 | 26 | 31 | 36 | 41 |
| % Released | | | | | | | | | | | | | | | | | |

Furthermore the invention allows for the combination of a SR/CR multiparticulate minicapsule with another SR/CR multiparticulate minicapsule and a IR multiparticulate minicapsule or other combinations thereof in varying ratio's of SR/CR:SR/CR:IR (%percent Example 30 + 31 + 29). A population of minicapsules from Example 30, Example 31 and Example 29 in varying ratio's as stated herein below were removed and blended in a suitable mechanical blender.The blended components were then filled into hard gelatine capsule to the required target strength.

Example 30 (45%) + Example 31 (45%) + Example 29 (10%)

Example 30 (50%) + Example 31 (30%) + Example 29 (20%)

Example 30 (30%) + Example 31 (60%) + Example 29 (10%)

### Example 32

| | |
|---|---|
| -- Micronised Nimodipine USP/EP | 500 grams |
| -- Fumaric Acid | 0-125 grams |
| -- Citric Acid | 0-125 grams |
| -- Talc | 5- 250 grams |
| -- Sodium Lauryl Sulphate | 0.125 grams |
| -- Sugar spheres (Non-Pareils) | 250 grams |
| -- Kollidon 30 (Povidone) | 50-150 grams |
| -- Eudragit RL | 5-15 grams |
| -- Eudragit RS | 35- 50 grams |
| -- Isopropyl Alcohol | as required |
| -- Acetone | as required |
| -- Diameter Multiparticulate Spheres | 1.50-1.80 mm |

The above example was produced by the multiparticulate layering process. This drug layering process is a well known and widely used technique in the drug delivery industry and is regularly used by formulation scientist to develop new delivery systems. The Nimodipine Applied Beads (IR) were manufactured as follows.

Nimodipine ,Fumaric Acid or Citric Acid or both, talc and sodium lauryl sulphate (active blend) were blended in a suitable Y-Cone blender. The active blend was applied using a suitable fluid bed system onto non-pareils using a suitable binder or adhering solution, such as Povidone from a suitable organic or aqueous solution such as isopropyl alcohol. The resultant immediate release beads were dried for approx 24 hours. The dried multiparticulate spheres were then screened and the appropriate fractions retained.

The applied beads (IR) were then further processed. A coating solution of a 6.25% solution of Eudragit RS (75-95% w/w) and Eudragit RL (5-25% w/w) dissolved in isopropyl alcohol/acetone mix was sprayed onto the applied beads using a suitable fluid bed system. Talc was added simultaneously via a mechanical feeder to prevent agglomeration. The result was a layered applied sphere with a rate-controlling polymer having a pre-determined dissolution profile.

The resultant coated spheres (SR) from this example were then blended with a percentage of the applied (IR) spheres. The blended spheres from the above were filled into hard gelatine capsules to a target strength.

Furthermore the above example could also be combined with other the examples listed above. The following combinations in varying % percent ratio's can also be produced to give a pre-determined dissolution profile :-

Example 29 + 30 + 31 + 32 or Example 30 + 31 + 32 or Example 31 + 32 and the like. The following ratio's are listed below as examples of the varying combinations that can be produced by removing a partial population of minicapsules from each of the above examples.

Example 29 (10%) + Example 30 (30%) + Example 31 (30%) + Example 32 (30%)

Example 30 (25%) + Example 31 (25%) + Example 32 (50%)

Example 31 (50%) + Example 32 (50%)

### Example 33

| Core Solution | |
|---|---|
| -- Nimodipine USP/EP | 500 grams |
| -- Low Viscosity MCT | 500 grams |

| Film Solution | |
|---|---|
| -- Gelatin | 590 grams |
| -- Sorbitol | 70 grams |
| -- Nimodipine USP/EP | 340 grams |
| -- Purified Water | 2290 grams |

| Polymer Coating Solution | |
|---|---|
| -- Eudragit S | as required |
| -- Isopropyl Alcohol/acetone | as required |
| -- Talc | as required |
| -- Minicapsule Diameter | 1.50-1.80 mm |

The above seamless minicapsules were manufactured in the same way as Example 29 with the following exceptions:-
1. The core solution was pre-treated with an Ultra Centrifugal Mill.
2. The film solution Nimodipine, was pre-treated with a High Pressure Homogeniser
3. The median solution was excluded this formulation.
4. Eudragit S was used as the polymer coat to provide an enteric coat with 0 drug release of up to 4 hours to the minicapsules, to target the drug release to the GIT and providing a pulsed release profile.

A percentage of the Enteric Coated Nimodipine minicapsules and a percentage of the coated minicapsules from Example 29 (as required) and a percentage of the uncoated minicapsules from Example 29 (as required) were blended as per in Example 29 and filled into suitable gelatin capsules to the target strength.

### Example 34

| Core Solution | |
|---|---|
| -- Nifedipine USP/EP | 100-400 grams |
| -- PEG 400 | 400-800 grams |

| Median Solution | |
|---|---|
| -- Low Viscosity MCT | 500-1000 grams |

| Film Solution | |
|---|---|
| -- Gelatin | 590 grams |
| -- Sorbitol | 70 grams |
| -- Nifedipine USP/EP | 100-400 grams |
| -- Purified Water | 1000-2500 grams |

| Polymer Coating Solution | |
|---|---|
| -- Eudragir S | as required |
| -- Isopropyl Alcohol/Acetone | as required |
| -- Talc | as required |
| -- Minicapsule Diameter | 1.50-1.80 mm |

The above seamless minicapsules were manufactured in the same way as Example 29 with the following exceptions: -
1. The Nifedipine core solution was pre-treated with an Ultra Centrifugal Mill.
2.The Nifedipine film solution, was pre-treated with a High Pressure Homogeniser.
3.Eudragit S was used as the polymer coat to provide an enteric coat with 0 drug release of up to 2-4 hours to the minicapsules, to target the drug release to the GIT and providing a pulsed release profile.

### Example 35

| Core Solution | |
|---|---|
| -- Micronised Nifedipine | 500-1000 grams |
| -- Gelatin | 500-3000 grams |
| -- Sorbitol | 0-200 grams |
| -- Purified Water | 4000-6000 grams |

The minicapsules in Example 35 were manufactured according to Examples 29 & 30 and filled into suitable hard gelatin capsules to the required target strength.

### Example 36

1. From Example 34 take a population of Immediate Release (IR) minicapsules.
2. Take a second population of Sustained Release (SR) minicapsules from Example 34
3. In the following ratio 5-25% Immediate Release (IR) and 75-95% Sustained Release (SR) minicapsules calculated by potency from Example 34 are blended using a suitable blender and encapsulated using suitable hard gelatin capsules into the target strengths.

### Example 37

| Core Solution | |
|---|---|
| -- Micronised Nimodipine USP/EP | 100-400 grams |
| -- PEG 400 | 400-800 grams |

| Median Solution | |
|---|---|
| Vegetable Oil or Mineral Oil | 0-1000 grams |

| Mucoadhesive Coating Solution | |
|---|---|
| -- Ethycellulose | 5-100 grams |
| -- PVP | 0.5-50 grams |
| -- Castor Oil | 0-50 grams |
| -- Magnesium Stearate | 0-50 grams |
| -- Acetone | as required |
| -- Isopropanol | as required |

| Film Solution | |
|---|---|
| -- Gelatin | 100-500 grams |
| -- Sumatriptan | 0-100 grams |
| -- Sorbitol | 0-50 grams |
| -- Purified water | 500- 3000 grams |

| Polymer Coating Solution | |
|---|---|
| -- Eudragit RL | 5% w/w |
| -- Eudragit RS | 95% w/w |
| -- Isopropyl Alcohol | as required |
| -- Acetone | as required |
| -- Talc | as required |
| -- Minicapsule Diameter | 0.5-1.80mm |

The Nimodipine Multiparticulate Seamless Minicapsules were manufactured according to freund Industrial Co. Ltd US Patent NO.5,882,680 (Seamless Capsule and Method of Manufacturing Same),as described in the Summary of the Invention Section. This example allows for the inclusion of the active ingredient in the Film Solution (gelatine layer) as also described in the Summary of the Invention Section.

To apply a mucoadhesive coating, a coating solution of 7% ethylcellulose,0.85% PVP and 1% magnesium stearate was dissolved in an isopropanol/acetone mixture. The solution was then sprayed coated onto the minicapsules using a suitable fluidised bed processor. Talc was used to prevent agglomeration of the minicapsules during the spray coating stage. The coated minicapsules were dried in an environmentally controlled drier at 40-50 deg.C for typically 12-24 hours.

In order to further coat the mucoadhesive coated seamless minicapsules, a coating solution of 6.25% Eudragit RL (5%w/w) and 6.25% Eudragit RS (95%w/w) dissolved in isopropyl alcohol/acetone mixture was sprayed coated onto the minicapsules using an automated fluidised bed processor. Talc was used to prevent agglomeration of the minicapsules during the spray coating stage. The coated minicapsules were further dried in an environmentally controlled drier at 40-SOdeg.C for typically 12-24 hours.

The Nimodipine seamless minicapsules produced in Example 36 were the encapsulated using suitable hard gelatine capsules into typically 30/60/90/120 or 180mg capsules or alternatively formats for rectal, vaginal or nasal administration.

It will be appreciated that any appropriate active ingredients mentioned may be formulated in a similar way to the preceding examples to provide formulations of the invention.

Some embodiments of the present invention will use one or more of the below ingredients in a multiparticulate capsule or sachet. It is noted that the following non-limiting lists illustrate exemplary ingredients that can be used with the present invention, including the broader subclasses and classes to which they belong.
1. *Enzymes* Alpha Galactosidase Amylase Bromelain Cellulase Papain Peptidase Protease Proteolytic Enzymes Superoxide Dismutase Trypsin
2. *Phospholipids* Lecithin Phosphatidyl Choline Phosphatidyl Serine
3. *Specialty Nutraceuticals* 5-Hydroxytryptophan Acetyl L-Carnitine Alpha Lipoic Acid Alpha-Ketoglutarates Bee Products Betaine Hydrochloride Bovine Cartilage Caffeine Cetyl Myristoleate Charcoal Chitosan Choline Chondroitin Sulfate Coenzyme Q10 Collagen Colostrum Creatine Cyanocobalamin (Vitamin B12) DMAE Fumaric Acid Germanium Sesquioxide Glandular Products Glucosamine HCL Glucosamine Sulfate HMB (Hydroxyl Methyl Butyrate) Immunoglobulin (Immune System Support) Lactic Acid L-Carnitine Liver Products Malic Acid Maltose-anhydrous Mannose (d-mannose) MSM Other Carnitine Products Phytosterols Picolinic Acid Pyruvate Red Yeast Extract SAMe Selenium Yeast Shark Cartilage Theobromine Vanadyl Sulfate Velvet Deer Antler Yeast.
4. *Herbal Oils* Aloe Vera Artichoke Oil Artichoke Oil Black Currant Seed Oil 14% GLA Black Currant Seed Oil 15% GLA Borage Oil 20% GLA Borage Oil 22% GLA Boswellia Serrata Oil CLA Conjugated Linolic Acid 75% min. Evening Primrose Oil 10% GLA Evening Primrose Oil 9% GLA Flax Seed Oil 50% ALA Garlic Oil Grape Seed Oil Guggul Lipid Oil Olive Leak Extract Oregano Oil Perilla Oil 60% ALA Pumpkin Seed Oil Pygeum Oil Rosehip Oil Rosemary Oil Saw Palmetto Oil Sterols Tocotrienol Palm Oil Walnut Oil Wheat Germ Oil Sesame Seed Oil Dill Seed Oil Clove Bud Oil Ginger Root Oil Cinnamon Leaf Oil Fennel Seed Oil Curcuma Longa Oil Cummin Seed Oil Celery Seed Oil Coriander Seed Oil Red Rasberry Seed Oil Cranberry Seed Oil Blackberry Seed Oil.
5. *Marine Oils* Cod Liver Oil (1000 A/100 D) Cod Liver Oil (2500A/250D) Fish Oil 30% EPA/20% DHA Fish Oil Concentrated Fish Oil Deodorized Marine Lipid Oil 18/12 Marine Lipid Oil 30/20 Marine Lipid Oil 36/24 Salmon Oil 18% EPA/12% DHA Squalene Oil (Shark)
6. *Other Oils* Alpha Lipoic Acid Cetyl Myristoleate CM Coenzyme Q10 . Lecithin Medium Chain Triglycerides MCT.
7. *Vitamins* Ascorbic Acid (Vitamin C) B Vitamins Biotin Fat Soluble Vitamins Folic Acid HCA (Hydroxycitric Acid) Inositol Mineral Ascorbates Mixed Tocopherols Niacin (Vitamin B3) Orotic Acid PABA (Para-Aminobenzoic Acid) Pantothenates Pantothenic Acid (Vitamin B5) Pyridoxine Hydrochloride (Vitamin B6) Riboflavin (Vitamin B2) Synthetic Vitamins Thiamine (Vitamin B 1) Tocotrienols Vitamin A Vitamin D Vitamin E Vitamin F Vitamin K Vitamin Oils Vitamin Premixes Vitamin-Mineral Premixes Water Soluble Vitamins
8. *Carotenoids* Apocarotenal Astaxanthin Beta-Carotene Canthaxanthin Carotenoids Lutein/I,utein Esters Lycopene Zeaxanthin
9. *Hormones* 7-Keto-DHEA Androstenedione DHEA Melatonin Nor-Androstenedione Pregnenolone Progesterone 19 Nor-4-Androstendiol 19 Nor-4-Androstenedione 19 Nor-5-Androstenediol 19 Nor-5-Androstendione 3-Indolebutyric Acid 4 Androstendiol 4 Androstendione 6 Furfurylaminopurene 6-Benzylaminopurine
10. *Minerals* Boron Calcium Chelated Minerals Chloride Chromium Coated Minerals Cobalt Copper Dolomite Iodine Iron Magnesium Manganese Mineral Premixes Mineral Products Molybdenum Other Minerals Phosphorus Potassium Selenium Sodium Specialty Minerals Trace Minerals Vanadium Zinc Malic Acid Pyruvate
11. *Probiotics* Acidophilus Bifido Bacteria Lactobacillus - both native wild-type and genetically modified probiotics.
12. *Proteins*/*Amino Acids* Amino Acids Betaine Casein Functional Soy Glutamic Acid L-Alanine L-Arginine L-Cysteine L-Glutamine L-Glycine L-Histidine L-Isoeucince L-Leucine L-Lysine L-Methionine L-Ornithine L-Phenylalaline L-Proline L-Taurine L-Threonine L-Tryptophan L-Tyrosine L-Valine N-Acetly-L-Cysteine Protein Soluble Soy Soy Protein Isolates Textured Soy Whey Protein Isolates
13. *Other Embodiments* Specialty Nutrients ATP Forskolin Sterol Esters Stanol EstersProbiotics LactoferinLutein Esters ZeaxanthinImmunoglobulins Ipriflavone Isoflavones Fructo-Oligo-Saccharides Inulin Huperzine A MelatoninMedicinal MushroomsBile Products Peptone Products Glandular Products Pancreatic Products Thyroid Products Ribose Probiotics Oleo Resins Dill Seed Oleo Resin Black Pepper Oleoresin Capsicum Oleoresin

The present invention can be used in cardiovascular treatments, for example hypertension, heart failure, and heart rhythm disorders. Also, the present invention can be used in immunology (e.g. transplant rejections, auto-immune disorders, etc.). The present invention can be used to treat neurological disorders (such as Parkinson's disease, dementia, stroke, epilepsy, and migraine headache, etc.), psychiatric disorders (schizophrenia, bipolar disease, depression, anxiety, ADHD/ADD, Addictions, etc.), infectious diseases (fungal, bacterial, viral (HIV), etc.), and in anesthesiology (induction anesthesia, local anesthesia). Furthermore, the present invention has application in endocrinology (cholesterol, diabetes, hormone replacement therapy, thyroid dysfunction, oral contraception, obesity, etc.), dermatology (onychomycosis, acne, rosaceae, psoriasis, etc.), rheumatology (arthritis, gout, osteoporosis/Osteomalacia), respiratory fields (asthma, emphysema, cystic fibrosis, etc.), gastro-intestinal fields (gastro-esophageal reflux disease, ulcer prophylaxis, crohn's disease, inflammatory bowel disease, etc.), chronic renal failure (vitamin and mineral replacement, blood pressure regulation, diabetes, depression, etc.), genito-urinary (enlarged prostate/BPH, overactive bladder, erectile dysfunction, feminine yeast infections, etc.) and hematology-oncology (thromboembolous, hermatopoeisis, neoplastic disease, nausea / vomiting).

The present invention further contemplates the use of any active ingredients or medicaments known in the art. The following non-limiting lists illustrate exemplary active ingredients or medicaments and the broader subclasses and classes to which they belong for use in this invention.
1. *Medicaments Acting on the Autonomic Nervous System* (Adrenergic c Medicaments, Cholinergic Medicaments,Direct Muscarinic Agonists Choline Esters) acetylcholine bethanechol carbachol methacholine Alkaloidsmuscarine pilocarpine
2. *Direct Nicotinic Agonist* nicotine
3. *Acetylcholinesterase Inhibitors* Acetylcholinesterase Inhibitors ("Reversible") - edrophonium neostigmine physostigmine Acetylcholinesterase Inhibitors ("Irreversible") - diisopropylflurorphosphate (DFP) echothiophate isoflurophate Muscarinic Antagonists Atropine ipratropium pirenzepine copolamine 2-PAM: Acetylcholinesterase Reactivator Pralidoxime (Protopam) {2-PAM}: peripheral acetylcholinesterase reactivator for certain phosphoryl-enzyme complexes
4. *Ganglionic Blockers* hexamethonium mecamylamine trimethaphan atecholamines dobutamine dopamine epinephrine isoproterenol norepinephrine
5. *Direct Adrenoceptor Agonist Medicaments* albuterol clonidine methoxamine oxymetazohne phenylephrine ritodrine salmeterol terbutaline
6. *Indirect-Acting Sympathomimetic Medicaments* amphetamine cocaine ephedrine, Pseudoephedrine tyramine
7. *Alpha-Adrenoceptor Antagonists Medicaments* doxazosin labetalol phenoxybenzamine phentolamine prazosin terazosin tolazoline trimazosin yohimbine
8. *Adrenoceptor Antagonist Medicaments* atenolol butoxaamine esmolol labetalolmetoprolol nadolol pindolol propranolol timolol
9. *Adrenergic Neuron Blocking Medicaments* guanethidine reserpine
10. *Cardiovascular System Disorders Medicaments* (ardiovascular testing and diagnosis Hypertension (HTN) Heart Failure Ischemic Heart Disease Myocardial Infarction Arrhythmias Isolated Diastolic Heart Failure and Cardiomyopathies Cardiac Transplantation Venous Thromboembolism Stroke Hyperlipidemia Peripheral vascular disease) - Diuretics carbonic-anhydrase inhibitors loop diuretics osmotic diuretics potassium sparing diuretics thiazide diuretics Anitiarrhythmic Medicaments Sodium Channel blocking agents isopyramide flecainide ibutilide lidocaine mexiletine moricizine procainamide propafenone quinidine tocainide Calcium Channel blocking agents bepridil diltiazem verapamil Adrenergic receptor antagonists propranolol adenosine amiodarone bretylium disopyramide esmolol sotalol CoA Reductase Inhibitors atorvastatin cerivistatin lovastatin pravastatin simvastatin Bile-acid sequestrants cholestyramine colestipol Fibric acids clofilbrate fenofibrate gemfibrozil niacin, nicotinic acid probucolacebutalol atenolol betaxolol bisoprolol carteolol clonidine labetalcl metoprolol penbutalol pindolol prazosin propranolol timolol Calcium Channel Antagonists amlodipine diltiazem felodipine isradipine nicardipine nifedipine nimodipinenisoldipine verapamil benazepril bepridil captopril enalapril fosinopril lisinopril moexipril quinapril ramipril losartan valasartan amiloride bumetanide chlorothalidone ethacrynic acid furosemide hydrochlorothiazide indapamide metolazone torsemide triamterene hydralazine minoxidil nitroprusside prazosin reserpine sotalol spironolactone terazosin Organic nitrates Calcium Channel Antagonists Adrenergic Receptor Antagonists amyl nitrite erythrityl tetranitrate isosorbide dinitrate nitroglycerin pentaerythritol tetranitrate phosphodiesterase (PDE) inhibitors anrinone milrinone carvedilol cardiac glycosides digitoxin digoxin diuretics ACE Inhibitors Dobutamine dopamine
11. *Respiratory System Disorders Medicaments* (Asthma Chronic Obstructive Lung Disease (COLD)/Chronic Obstructive Pulmonary Disease (COPD) Acute Respiratory Distress Syndrome (ARDS) Drug-Induced Pulmonary Disease Cystic Fibrosis)Corticosteroids beclomethasone betamethasone cortisone dexamethasone fluticasone (Flovent/Flonase) hydrocortisone methylprednisolone prednisolone prednisone triamcinolone sympathomimetics albuterol salmeterol muscarinic antagonists ipratropium leukotriene pathway inhibitors montelukast zafirtukast mast cell stabilizers cromolyn methylxanthines theophylline aminophylline Dnase
12. Gastrointestinal System Disorders (Gastro-esophageal Reflux Disease (GERD) Peptic Ulcer Disease Inflammatory Bowel Disease Nausea and Vomiting Diarrhea, Constipation, Irritable Bowel Disease (IBD) Drug Induced Liver Disease Pancreatitis Viral Hepatitis Liver Transplantation) - Histamine-2 receptor antagonists famotidine nizatidine pantoprazole rabeprazole ranitidineProton Pump Inhibitors esomeprazole lansoprazole omeprazole anticholinergics antihistamines (Histamine-i receptor antagonists) dopamine antagonists prokinetic gastric stimulant serotonin 5HT.sub.3 receptor antagonists dolasetron granisetron ondansetron hydroxyzine corticosteroids benzodiazepines cannabinoids Prokinetic gastric stimulants cisapride metoclopramideLaxativesSaline laxatives magnesium salts sodium salts irritant/stimulant medicaments cascara senna phenolphthalein bisacodylcasanthranol castor oil methylcellulose psyllium polycarbophil lubricant mineral oil surfactants docusate glycerin lactulose Anti -diarrheal medicaments diphenoxylate atropine diphenoxin loperamide bismuth lactobacillus mesalamine olsalazine
13. *Renal System Disorders Medicaments* (Acute Renal Failure Progressive Renal Failure/Chronic Renal Failure Neurologic System Disorders Multiple Sclerosis and inflammatory polyneuropathies Epilepsy Parkinson's disease and Movement Disorders Pain management Headache Amyotrophic Lateral Sclerosis Anti-Epileptic) carbamazepine divalproex sodium felbamate gabapentin lamotrigine oxcarbazepine phenytoin topiramate zonisamide Serotonin SHT.sub.ld receptor agonists almotriptan frovatriptan naratriptan rizatriptan sumatriptan zolmitriptan ergot alkaloids dihydroergotamine isometheptine/dichlorophenazone caffeine pizotifen benzodiazepines alprazolam clonazepam clorazepate diazepam flumazenil antagonist lorazepam midazolam triazolam barbiturates/Anesthetics pentobarbital phenobarbital thiopental non-depressant anxiolytic buspirone
14. *Treatment of Alcoholism Medicaments* disulfiram
15. *Pain ManagementMedicaments* Opioids Opioid Peptides beta-endorphin dynorphin enkephalins Agonists codeine etorphine fentanyl hydrocodeine hydromorphone meperidine methadone morphine oxycodone propoxyphene buprenorphine dezocine nalbuphine pentazocine naloxone Non-opiate acetaminophen tramadol
16. *Anti-Parkinsonism Medicaments* levodopa carbidopa bromocriptine pergolide amantadine selegiline anticholinergic agents dopamine Agonists pramipexole ropinirole COMT inhibitors entacapone tolcapone Anti-Spasticity Medicaments baclofen botulinum toxin type A carisoprodol chlorphenesin chlorzoxazone cyclobenzaprine dantrolene diazepam metaxalone methocarbamol orphenadrine tizanidine
17. *Psychiatric System Disorders* (Childhood psychiatric disorders Attention Deficit Hyperactivity Disorder (ADHD)/Attention Deficit Disorder (ADD) Eating disorders Alzheimer's disease and Dementia Disorders Substance abuse and Addictive Disorders alcohol, tobacco and caffeine abuse Schizophrenia Depressive disorders Bipolar disorders Anxiety disorders Obsessive-Compulsive disorders Sleep disorders)Psychostimulant medicaments amphetamine mixed salts dextroamphetamine methylphenidate Antipsychotic Medicaments Phenothiazine type chlorpromazine fluphenazine Thioxanthene type thiothixene Butyrophenone type haloperidol Dibenzodiazepine type clozapine Thienobenzodiazepine type olanzapine quetiapine Antidepressant Medicaments Tricyclic antidepressants amitriptyline clomipramine also a SSRI desipramine doxepin imipramine maprotiline nortriptytine protriptyline Monoamine oxidase inhibitors (MAO-I's) clorgyline (specific for MAO type A) isocarboxazid phenelzine tranylcypromine Second Generation Medicaments (not including SSRIs) amoxapine bupropion netazodone trazodone Serotonin-Specific Reuptake Inhibitors (SSRIs) citalopram clomipramine escitalopram fluoxetine fluvoxamine paroxetine sertraline lithium mirtazapine venlafaxine
18. *Anti-Anxiety Agents* barbiturates benzodiazepines buspirone chloral hydrate doxepin hydroxyzine sedative-hypnotics serotonin reuptake inhibitors
19. *Anti-Demential Medicaments* cholinesterase inhibitors donepezil galantamine rivastigmine tacrine
20. *Endoctimologic System Disorders Medicaments* (Diabetes mellitus Thyroid disorders Adrenal Gland disorders Pituitary Gland disorders ACTH Adrenal androgens Adrenocortical Function Antagonists Mineralocorticoid antagonists)-Anti-Diabetic Medicaments Insulin Sulfonylureas acetohexamide chlorpropaamide glimepiride glipizide glyburide tolazarnide tolbutamide Biguanides metformnin Alpha-glucosidase Inhibitors acarbose miglitol
   Thiazolidinedione Derivatives pioglitazone rosiglitazonetroglitazone Thyroid Disorder Medicaments Levothyroxine Liothyronine Liotrix Hypothalamic and Pituitary Gland Medicaments bromocriptine chorionic gonadotropin (hCG) corticotropin generic (ACTH) cosyntropin desmopressin gonadorelin acetate (GnRH) gonadorelin hydrochloride (GnRH) goserelin acetate growth hormone histrelin leuprolide menotropins (hMG) natarelin octreotide oxytocin pergolide protirelin sermorelin (GHRH) somatrem somatropin thyrotropin (TSH) urofollitropin vasopressin
21. *Gynecologic System and Obstetric Conditions Medicaments* (Pregnancy and Lactation Infertility Contraception Menstruation-related disorders Endometriosis Hormone Replacement Therapy (HRT) )Conjugated estrogens desogestrel di-norgestrel ethinyl diacetate ethinyl estradiol levonorgestrel medroxyprogesterone norethindrone norgestimate progesterone
22. *Urologic System Disorders Medicaments* (Erectile Dysfunction Benign Prostatic Hypertrophy Urinary Incontinence)apomorphine alprostadit phosphodiesterase (PDE-5) inhibitors sildenafil tadalafil vardenafil tolterodine tamulosin yohimbine
23. *Immunologic System Disorders Medicaments* (Systemic Lupus Erythematosus and other Collagen-vascular diseases Allergic and pseudo-allergic drug reactions Bone and Joint System Disorders Osteoporosis and Osteomalacia Rheumatoid Arthritis Osteoarthritis Gout and hyperuricemia ) - Medicaments used in the Control of Inflammation Non-steroidal antiinflammatory drugs (NSAIDs) aspirin diclofenac diflusnisal etodolac fenoprofen flubiprofen ibuprofen indomethacin ketoprofen ketorolac meclofenamate nabumetone naproxen oxaprozin phenylbutazone piroxicam salicytate sulindac tolmetin
   Cyclocxygenase-2 inhibitors (COX-2) celecoxib rofecoxib Arthritis and Gout Medicaments allopurinol chloroquine colchicine enbrel Glucocorticoids Gold methotrexate NSAIDs Penicillamine alendronate raloxifene
24. *Disorders of the Eyes, Ears, Nose, and Throat Systems Medicaments* (Glaucoma Allergic rhinitis) Histamine-1 receptor antagonists brompheniramine cetirizine chlorpheniramine clemastine cyproheptadine dimenhydrinate diphenhydramine doxylamine fexofenadine loratidine Sympathomimetic medicaments pseudoephedrine
25. *Dermatologic System Disorders Medicaments* (Acne Psoriasis Rosacea and pigmentation disorders Hematologic System Disorders Hematopoeisis Anemias Coagulation disorders Sickle-cell anemia Drug-induced hematologic disorders)
26. *Coagulation Disorders Medicaments -* aspirin clopidogrel fibrinolytic inhibitors fibrinolytics glycoprotein (GP) IIb/IIIa antagonists/monoclonal antibodies abciximab eptifibatide tiofibran heparin low-molecular weight heparins Plasma fractions-blood factors ticlopidine vitamin K warfarin
*27. Vaccines, toxoids, and other immunobiologics*
28. *Antibiotics* Penicillins amoxicillin ampicillin benzathine Penicillin G benzyl Penicillin carbenicillin cloxacillin dicloxacillin methicillin mezlocillin nafcillin oxacillin phenoxymethyl Penicillin piperacillin ticarcillin Cephalosporins 1st generation: cefazolin cephalexin cephatothin 2nd generation: cefaclor (Ceclor) cefoxitin (Mefoxin) cefpodoxime (Vantin) cefuroxime (Zinacef, Ceftin) loracarbef (Lorabid) 3rd generation: cefoperazone cefotaxime (Claforan) cefotetan ceftazidime (Fortax, Taxidime, Tazicef) ceftriaxone (Rocephin) veftizoxime (Cefizox) 4th generation: cefepime Other beta-Lactams aztreonam (Azactan) clavulanic acid imipenem (Primaxin) meropenem (Merrem IV) sulbactam Other Cell-Wall Synthesis Inhibitors bacitracin cycloserine fosfomycin vancomycin
29. *Agents Which Affect Cell Membranes* Polymixins Colistimethate Potymyxin B
30. *Protein Synthesis Inhibitors* Aminoglycosides amikacin gentamicin kanamycin neomycin netilmicin streptomycin tobramycin Tetracyclines demeclocycline doxycycline doxycyclrnue tetracycline Macrolides azithromycin clarithromycin erythromycin esters erythromycin Other Protein Synthesis Inhibitors Chloramphenicol (Chloromycetin) Clindamycin (Cleocin) Spectinomycin (Trobicin) Inhibitors of Folate-Dependent Pathways co-trimoxazole silver Sulfadiazine sodium Sulfacetamide sulfamethoxazole (Gantanol) sulfasalazine (Azulfidine) (Salicylazosulfapyridine) sulfisoxazole (Gantrisin) sulfonamides Dihydrofolate Reductase Inhibitor trimethoprim
31. *DNA Gyrase Inhibitors* ciprofloxacin gatifloxacin levofloxacin lomefloxacin nalidixic acid ofloxacin
*32. Urinary Tract Antiseptics* nitrolurantoin
33. *Antimyobacterial Agents* First-line anti-TB medicaments ethambutol isoniazid (INI-I) pyrazinamide rifampin (Rimactane) streptomycin Second-line anti-TB medicaments capreomycinA cycloserine dapsone ethionamide para-aminosalicylic acid
34. *AntiFungal Agents* amphotericin B clotrimazole fluconazole flucytosine griseofulvin itraconazole ketoconazole miconazole nystatin
35. *AntiParasitic Agents* Antimalarials chloroquine mefloquine primaquine pyrimethamine-sulfadoxine Anti protozoals metronidazole pentamidine isethionate pyrimethamine-sulfonamide trimethoprim sulfamethoxazole
36. *Antihelminthic Medicaments*mebendazole praziquantel pyrantel pamoate thiabendazole
37. *Antiviral Medicaments* acyclovir didanosine foscarnet ganciclovir ribavirin rimantadine stavudine valacyclovir vidarabine zalcitabine zidovudine
38. *Protease inhibitors* indinavir ritonavir saquinavir
39. *Oncologic and Immunological Disorders Medicaments* (Breast Cancer Lung Cancer Colorectal Cancer Prostate Cancer Malignant Lymphomas Ovarian Cancer Acute Leukemias Chronic Leukemias Melanoma and other Skin Cancers Hematopoeitic Stem Cell Transplantation) - Anti-Neoplastic Medicaments Alkylating Agents busulfan carboplatin carmustine cisplatin cyclophosphamide ifofamide lomustine mechlorethamine meiphalan procarbazine thiotepa Antimetabolites folic acid Antagonist methotrexate Purine Antagonists 6-mercaptopurine 6-thioguanine Pyrimidine Antagonists cytarabine fluorouracil Hormonal Agents: Hormones diethylstilbestrol estrogens prednisone Modulation of Hormone Release & Action Aminoglutethimide leuprolide acetate tamoxifen Plant Alkaloids Vinca Alkaloids vinblastine vincristine Podophyllotoxins Etoposide (VP-16) Others- docetaxel paclitaxel Antibiotics - bleomycin dactinomycin daunorubicin doxorubicin mitomycin Other Anti-neoplastic Medicaments amsacrine azathioprine capecitabine chlorambucil cyclosporine 5 gemcitabine hydroxyurea mitotane mitoxantrone pamidronate
40. *Immunosuppressant Medicaments* 15-desoxyspergualin corticosteroids cyclosporine Interferons Interleukins mycophenolate mofetil sirolimus (rapamycin) tacrolimus thalidomide
41. *Nutritional Disorders Medicaments* (Malnutrition, vitamin and mineral deficiencies Enteral Nutrition Obesity) orlistat appetite suppressants sympathomimetic stimulants amphetamine stimulants Mineral supplementation calcium ion iodine iron magnesium ion phosphorous potassium ion selenium sodium ion zinc Fat-soluble vitamins vitamin A vitamin D vitamin E vitamin K Water-soluble vitamins vitamin C thiamine (vitamin B1) riboflavin (vitamin B2) niacin (vitamin B3) pyridoxine (vitamin B6) folate cyanocobalamin (vitamin B 12)
42. *Medicaments used to Alleviate Symptoms of Allergic Rhinitis Upper Respiratory Symptoms, Cough, Mild Aches and Pains* Nasal Decongestants ephedrine phenylephrine phenylpropanolamine pseudoephedrine Antihistamines (Histamine-1 receptor antagonists)
43. *Antitussive agents* benzonatate codeine dextromethorphan Expectorants guaifenesin iodinated glycerol terpin hydrate Xanthines aminophylline caffeine dyphylline theophylline Pain relievers narcotic agonists NSAIDS acetam inophen
44. *Dietary Supplements* Arnica Bilberry Black Cohosh Cat's claw Chamomile Echinacea Evening Primrose Oil Fenugreek Flaxseed Feverfew Garlic Ginger root Ginkobiloba Goldenrod Hawthorn Kava-Kava Licorice Milk thistle Psyllium Rauwolfia Senna Soybean St. John's wort Saw palmetto Turmeric Valerian
45. *Therapeutic Proteins and Biotechnology Medicaments*
46. *Additional Agents* Norvasc, Neurontin, Paxil, Augmentin, Propecia, Lamisil, Lescol, bisphosphonate abacavir sulfate acetazolamide acetylsalicylic acid albendazole allopurinol amiloride hydrochloride amitriptyline hydrochloride artemether atropine sulfate benznidazole biperiden hydrochloride chloroquine phosphate chlorpheniramine maleate chlorpromazine hydrochloride cimetidine ciprofloxacin hydrochloride clofazimine clomiphene citrate clomipramine hydrochloride cloxacillin sodium codeine phosphate dapsone didanosine diethylcarbamazine citrate digoxin diloxanide furoate DL-methionine Doxycycline Efavirenz ergometrine maleate ergotamine tartrate erythromycin ethyl succinate ethambutol hydrochloride ethosuximide ferrous sulfate alendronate sodium amlodipine besylate amphetamineatorvastatin calcium benazepril hydrochloride bisoprolol fumarate bupropion hydrochloride carbidopa cefprozil cetirizine hydrochloride citalopram hydrobromide clindamycin hydrochloride clonidine hydrochloride clopidogrel bisulfate cyclobenzaprine hydrochloride desloratadine digoxin diltiazem hydrochloride doxazosin mesylate doxycycline enalapril maleate fexofenadine hydrochloride fluoxetine hydrochloride folic acid fosinopril sodium hydrocodone bitartrate hydrocodone hydroxyzine hydrochloride indinavir irbesartan isosorbide mononitrate lamivudine levothyroxine sodium lopinavir loratadine losartan potassium meclizine hydrochloride medroxyprogesterone acetate meperidine metformin hydrochloride methylphenidate hydrochloride methylprednisolone metoclopramide hydrochloride) minocycline hydrochloride montelukast sodium naproxen sodium nelfinavir nevirapine niclosamide nicotinamide nifurtimox nitrofurantoin nortriptyline hydrochloride oxybutynin chloride oxycodone hydrochloride paracetamol paroxetine hydrochloride penicillin V potassium phenytoin sodium pioglitazone hydrochloride prednisolone primaquine phosphate pravastatin sodium prednisolone promethazine hydrochloride promethazine fumarate propylthiouracil pyrantel embonate pyridostigmine bromide raloxifene hydrochloride ranitidine hydrochloride rifampicin risedronate sodium risperidone rosiglitazone maleate salbutamol sulfate saquinavir mesylate sertraline hydrochloride sildenafil citrate sulfadiazine sumatriptan succinate tamoxifen citrate tamsulosin hydrochloride temazepam terazosin hydrochloride timolol maleate tolterodine tartrate tramadol hydrochloride trazodone hydrochloride triclabendazole valacyclovir hydrochloride valdecoxib valproic acid valsartan venlafaxine hydrochloride verapamil hydrochloride warfarin sodium zolpidem tartrate.
47. Nucleic acids, single and double stranded DNA, modified DNA, RNA, mRNA, SiRNA, gene vectors, antisense oligonucleotides, decoy oligonucleotides, ribozymes, single and double stranded aptamers,

The minicapsules may contain various combinations of active ingredients. Such combinations are described in our co-pending PCT application filed September 27, 2005, and entitled "Combination Products", the entire contents of which are herein incorporated by reference.

This invention is not limited to the embodiments hereinbefore described which may be varied in detail.

Specific embodiments of the invention are illustrated by way of the following numbered paragraphs.
1. A formulation comprising a plurality of seamless minicapsules, having a diameter of from 0.5 mm to 5 mm the minicapsules having a core containing an active entity and an encapsulating body, the active entity being in the form of any one or more of:-
   a microemulsion,
   a nanoemulsion,
   a self-emulsifying delivery system,
   a self-microemulsifying delivery system,
   a biostable perflurocarbon formulation,
   a complex with cyclodextrin (and the like),
   liposomes,
   hydrogel,
   lymphatic targeted delivery system,
   liquid bi-layers
   wax
   an aqueous system
   emzaloid
   natural plant extract.
2. A formulation of paragraph 1 wherein at least some of the minicapsules have at least one coating to control the time and/or location of the release of the active entity.
3. A formulation of paragraph 2 wherein the coated seamless minicapsules have a diameter of from 0.5 mm to 5.0 mm.
4. A formulation of paragaph 2 or 3 wherein the coated seamless minicapsules have a diameter of from 1.2 mm to 2.0 mm.
5. A formulation of any of paragraphs 2 to 4 wherein the coated seamless minicapsules have a diameter of from 1.4 mm to 1.8 mm.
6. A formulation of any of paragraphs 1 to 5 wherein at least one coating is an immediate release coating.
7. A formulation of any of paragraphs 1 to 6 wherein at least one coating is a sustained release coating.
8. A formulation of any of paragraphs 1 to 7 wherein the coating comprises a sustained release and an immediate release coating.
9. A formulation of any of paragraphs 1 to 8 wherein at least one coating is an enteric coating.
10. A formulation of any of paragraphs 1 to 9 wherein at least one coating is a bioadhesive coating.
11. A formulation of paragraph 10 wherein the bioadhesive coating is a mucoadhesive coating.
12. A formulation of any of paragraphs 1 to 11 wherein the minicapsule comprises a buffer layer.
13. A formulation of any of paragraphs 1 to 12 wherein the minicapsule is formed from a core solution containing an active ingredient, and an encapsulating solution which forms, on setting, the encapsulating medium.
14. A formulation of paragraph 13 wherein the encapsulating solution contains an active ingredient.
15. A formulation of paragraph 14 wherein the active ingredient contained in the encapsulating solution is the same as the active ingredient in the core solution.
16. A formulation of paragraph 14 wherein the active ingredient contained in the encapsulating solution is different from the active ingredient in the core solution.
17. A formulation of any of paragraphs 14 to 16 wherein the active ingredient contained in the encapsulating solution is in a micronised or nanonized particle form.
18. A formulation of any of paragraphs 1 to 12 wherein the minicapsule is formed form a solution containing the encapsulating medium and an active ingredient.
19. A formulation of any of paragraphs 1 to 18 wherein the active ingredient contained in the encapsulating solution is in a micronised or nanonized particle form.
20. A formulation of any of paragraphs 1 to 19 comprising at least two different populations of minicapsules.
21. A formulation of paragraph 20 wherein one population of minicapsules comprises minicapsules with one rate-controlling coating and another population of minicapsules comprises minicapsules with a second rate-controlling coating.
22. A formulation of paragraph 21 wherein one population of minicapsules has an immediate release coating and the other population of minicapsules has a sustained or controlled release coating.
23. A formulation of any of paragraphs 20 to 22 wherein one population of minicapsules does not have a coating.
24. A formulation of any of paragraphs 20 to 23 wherein one population of minicapsules contains a first active ingredient and another population of minicapsules contains a second active ingredient.
25. A formulation of any of paragraphs 1 to 24 comprising a capsule containing a plurality of minicapsules.
26. A formulation of paragraph 25 wherein the capsule contains another entity.
27. A formulation of paragraph 25 wherein the other entity is in a liquid, powder, semi-solid, solid or gaseous form.
28. A formulation of paragraphs 26 or 27 wherein the other entity comprises an active entity.
29. A formulation of any of paragraphs 1 to 24 comprising a tablet or pellet containing a plurality of minicapsules.
30. A formulation of paragraph 29 wherein the tablet or pellet contains another entity.
31. A formulation of paragraph 30 wherein the other entity is an active entity.
32. A formulation comprising a plurality of seamless minicapsules, the minicapsules containing an active entity in a solid and/or semi-solid form and an encapsulating medium, the seamless minicapsules having a diameter of from 0.5 mm to 5 mm.
33. A formulation of paragraph 32 wherein at least some of the minicapsules have at least one coating to control the time and/or location of the release of the active entity.
34. A formulation of paragraph 33 wherein the coated seamless minicapsules have a diameter of from 0.5 mm to 5.0 mm.
35. A formulation of paragraphs 33 to 34 wherein the coated seamless minicapsules have a diameter of from 1.2 mm to 2.0 mm.
36. A formulation of any of paragraphs 33 to 35 wherein the coated seamless minicapsules have a diameter of from 1.4 mm to 1.8 mm.
37. A formulation of any of paragraphs 32 to 36 wherein at least one coating is an immediate release coating.
38. A formulation of any of paragraphs 32 to 37 wherein at least one coating is a sustained release coating.
39. A formulation of any of paragraphs 32 to 38 wherein the coating comprises a sustained release and an immediate release coating.
40. A formulation of any of paragraphs 32 to 39 wherein at least one coating is an enteric coating.
41. A formulation of any of paragraphs 32 to 40 wherein at least one coating is s a bioadhesive coating.
42. A formulation of paragraph 41 wherein the bioadhesive coating is a mucoadhesive coating.
43. A formulation of any of paragraphs 32 to 42 comprising a buffer layer.
44. A formulation of any of paragraphs 32 to 43 wherein the minicapsule is formed from a core solution containing an active ingredient, and an encapsulating solution which forms, on setting, the encapsulating medium.
45. A formulation of paragraph 44 wherein the encapsulating solution contains an active ingredient.
46. A formulation of paragraph 45 wherein the active ingredient contained in the encapsulating solution is the same as the active ingredient in the core solution.
47. A formulation of paragraph 45 wherein the active ingredient contained in the encapsulating solution is different from the active ingredient in the core solution.
48. A formulation of any of paragraphs 45 to 47 wherein the active ingredient contained in the encapsulating solution is in a micronised or nanonized particle form.
49. A formulation of any of paragraphs 32 to 43 wherein the minicapsule is formed form a solution containing the encapsulating medium and an active ingredient.
50. A formulation of any of paragraphs 32 to 49 wherein the active ingredient contained in the encapsulating solution is in a micronised or nanonized particle form.
51. A formulation of any of paragraphs 32 to 50 comprising at least two different populations of minicapsules.
52. A formulation of paragraph 51 wherein one population of minicapsules comprises minicapsules with one rate-controlling coating and another population of minicapsules comprises minicapsules with a second rate-controlling coating.
53. A formulation of paragraph 52 wherein one population of minicapsules has an immediate release coating and the other population of minicapsules has a sustained or controlled release coating.
54. A formulation of any of paragraphs 51 to 53 wherein one population of minicapsules does not have a coating.
55. A formulation of any of paragraphs 51 to 54 wherein one population of minicapsules contains a first active ingredient and another population of minicapsules contains a second active ingredient.
56. A formulation of any of paragraphs 32 to 55 comprising a capsule containing a plurality of minicapsules.
57. A formulation of paragraph 56 wherein the capsule contains another entity.
58. A formulation of paragraph 56 wherein the other entity is in a liquid, powder, semi-solid, solid or gaseous form.
59. A formulation of paragraphs 57 or 58 wherein the other entity comprises an active entity.
60. A formulation of any of paragraphs 32 to 55 comprises a tablet or pellet containing a plurality of minicapsules.
61. A formulation of paragraph 60 wherein the tablet or pellet contains another entity.
62. A formulation of paragraph 61 wherein the other entity is an active entity.
63. A formulation comprising a plurality of seamless minicapsules comprising a plurality of particles containing an active entity dispersed in an encapsulating body, the seamless minicapsules having a diameter of from 0.5 mm to 5 mm.
64. A formulation of paragraph 63 wherein at least some of the minicapsules have at least one coating to control the time and/or location of the release of the active entity.
65. A formulation of paragraph 64 wherein the coated seamless minicapsules have a diameter of from 0.5 mm to 5.0 mm.
66. A formulation of paragraphs 64 or 65 wherein the coated seamless minicapsules have a diameter of from 1.2 mm to 2.0 mm.
67. A formulation of any of paragraphs 64 to 66 wherein the coated seamless minicapsules have a diameter of from 1.4 mm to 1.8 mm.
68. A formulation of any of paragraphs 63 to 67 wherein at least one coating is an immediate release coating.
69. A formulation of any of paragraphs 63 to 68 wherein at least one coating is a sustained release coating.
70. A formulation of any of paragraphs 63 to 69 wherein the coating comprises a sustained release and an immediate release coating.
71. A formulation of any of paragraphs 63 to 70 wherein at least one coating is an enteric coating.
72. A formulation of any of paragraphs 63 to 71 wherein at least one coating is a bioadhesive coating.
73. A formulation of paragraph 72 wherein the bioadhesive coating is a mucoadhesive coating.
74. A formulation of any of paragraphs 63 to 73 wherein the minicapsule is formed form a solution containing the encapsulating medium and an active ingredient.
75. A formulation of any of paragraphs 63 to 74 wherein the active ingredient contained in the encapsulating medium is in a micronised or nanonized particle form.
76. A formulation of any of paragraphs 63 to 75 comprising at least two different populations of minicapsules.
77. A formulation of paragraph 76 wherein one population of minicapsules comprises minicapsules with one rate-controlling coating and another population of minicapsules comprises minicapsules with a second rate-controlling coating.
78. A formulation of paragraph 77 wherein one population of minicapsules has an immediate release coating and the other population of minicapsules has a sustained or controlled release coating.
79. A formulation of any of paragraphs 76 to 77 wherein one population does not have any rate-controlling coating.
80. A formulation of any of paragraphs 76 to 78 wherein one population of minicapsules contains a first active ingredient and another population of minicapsules contains a second active ingredient.
81. A formulation of any of paragraphs 32 to 80 comprising a capsule containing a plurality of minicapsules.
82. A formulation of paragraph 81 wherein the capsule contains another entity.
83. A formulation of paragraph 82 wherein the other entity is in a liquid, powder, semi-solid, solid or gaseous form.
84. A formulation of paragraphs 82 or 83 wherein the other entity comprises an active entity.
85. A formulation of any of paragraphs 63 to 84 comprises a tablet or pellet containing a plurality of minicapsules.
86. A formulation of paragraph 85 wherein the tablet or pellet contains another entity.
87. A formulation of paragraph 86 wherein the other entity is an active entity.
88. A formulation comprising a plurality of seamless minicapsules having at least two populations selected from:-
   a first minicapsule population in which the minicapsules comprise a core containing an active ingredient and an encapsulating medium, the minicapsules having a diameter of from 0.5 mm to 5 mm;
   a second minicapsule population in which the minicapsules comprise a plurality of particles containing an active entity dispersed in an encapsulating medium, the minicapsules having a diameter of from 0.5 mm to 5 mm; and
   a third micro or mini particles population in which the minicapsules comprise an inert core and at least one layer around the core, the layer containing an active ingredient.
89. A formulation of ant of paragraphs 1 to 88 wherein at least some of the minicapsules are provided with a bioadhesive such as a mucoadhesive.
90. A formulation of paragraph 89 wherein the bioadhesive comprises from 0% to 10% by weight of one or more of the following polymer classes: - polyacrylates; polyanhydrides; chitosans; carbopols; cellulose; methylcellulose; methylated deoxycellulose (m-doc^{™}), lectins.
91. A formulation of paragraph 89 wherein the bioadhesive comprises from 0% to 10% by weight of one or more of the following thiolated or otherwise derivatised polymers:- polyacrylates; polyanhydrides; chitosans; carbopols; cellulose; methylcellulose; methylated deoxycellulose (m-doc^{™}), lectins.
92. A formulation of any of paragraphs 89 to 91 wherein the bioadhesive comprises a coating.
93. A formulation of paragraphs 89 to 92 wherein the bioadhesive is incorporated into a part or layer of the minicapsule.
94. A formulation of paragraph 93 wherein the bioadhesive is incorporated into a rate-controlling layer.
95. A formulation of paragraphs 93 or 94 wherein the bioadhesive is incorporated into the encapsulating medium
96. A formulation of any of paragraphs 1 to 95 wherein at least some of the minicapsules have a layer such as an outer layer which is divided into at least two parts.
97. A formulation of paragraph 96 wherein the parts are of the same composition.
98. A formulation of paragraph 96 wherein at least some of the parts have different composition.
99. A formulation substantially as hereinbefore described with reference to the examples.
100. A formulation of any of paragraphs 1 to 99 wherein the minicapsules are filled into hard gelatin capsules.
101. A formulation of any of paragraphs 1 to 99 wherein the minicapsules are filled into a sachet.
102. A formulation of any of paragraphs 1 to 99 wherein the minicapsules are suspended in oil as a lubricant.
103. A formulation of any of paragraphs 1 to 99 wherein the minicapsules are contained within a wide gauge syringe that is compatible with tube delivery.
104. A formulation of any of paragraphs 1 to 99 wherein the minicapsules are in the form of a sprinkle.
105. A formulation of any of paragraphs 1 to 99 wherein the minicapsules are formulated as a suppository for rectal or vaginal administration.
106. A formulation of any of paragraphs 1 to 99 wherein the minicapsules are formulated for nasal administration.

## Claims

1. A seamless minicapsule containing a pharmaceutically active ingredient solubilised or dispersed in a pharmaceutically acceptable liquid phase, and obtainable by a process which utilises surface tension wherein two different solutions which do not dissolve with each other or hardly dissolve with each other contact each other, the process comprising ejecting through a nozzle having a single orifice, and into a cooling solution a shell/core mixed suspension, which outer shell solution is gelled in the cooling solution, wherein the shell part is a gelatin-based solution and the core part is a hydrophobic solution or suspension, the hydrophobic solution or suspension comprising a pharmaceutically active ingredient solubilised or dispersed in a pharmaceutically acceptable liquid phase, the shell of the minicapsule being coated with a coating selected from a polymer coating comprising an ethylcellulose, a small intestinal-specific releasing enteric coating, and a colon-specific releasing coating.

2. A minicapsule of claim 1 which has having a diameter of from 1.2mm to 3mm and optionally a diameter of less than 2mm.

3. A minicapsule of claim 1 or claim 2 selected from:
a) minicapsules wherein the shell has a polymer coating comprising ethylcellulose; and/or
b) minicapsules wherein the core part is a hydrophobic solution.

4. A seamless minicapsule of any preceding claim when in the form of a solid oral dosage form comprising a multiplicity of such seamless minicapsules.

5. A formulation comprising a plurality of seamless minicapsules, having a diameter of from 0.5 mm to 5 mm the minicapsules having a core containing an active entity and an encapsulating body being a gelled or solidified encapsulating solution of a member selected from the group consisting of gelatin, chitosan, gellan gum, carrageenan, xanthan gum, phthalated gelatin, succinated gelatin and combinations thereof, the active entity being in a form selected from:-
• a microemulsion,
• a nanoemulsion,
• liposomes,
• liquid bi-layers
• emzaloid
• micelles entrapping both hydrophobic and hydrophilic drugs in aqueous and lipid vehicles respectively
• polymeric micelles
• a core containing an active entity solubilised in an acceptable solvent that is solid or semi-solid at ambient temperature
• a water-free wax formulation optionally comprising cells or a vaccine,
and/or the active entity being a live, attenuated or killed mammalian, bacterial or viral cell culture.

6. A formulation as claimed in claim 5 wherein at least some of the minicapsules have at least one coating to control the time and/or location of the release of the active entity.

7. A formulation of claim 5 or claim 6 wherein at least one coating is an enteric coating.

8. A formulation of any of claims 5 to 7 wherein the encapsulating body contains an active ingredient, optionally wherein the active ingredient is in micronised or nanonised particle form.

9. A formulation of any of claims 5 to 8 comprising a capsule containing a plurality of minicapsules.

10. A formulation of any of claims 5 to 9 wherein the encapsulating body is coated with either or both of a small intestinal-specific releasing enteric coating and/or a colon-specific releasing coating.

11. A formulation of any of claims 5 to 10 wherein the encapsulating solution is a solution of gelatin.

12. A formulation of any of claims 5 to 11 wherein the minicapsules are obtainable by a process which utilises surface tension wherein two different solutions which do not dissolve with each other or hardly dissolve with each other contact each other, the process comprising ejecting into a cooling solution a core solution and an encapsulating solution, which encapsulating solution is gelled in the cooling solution, wherein either:
a) an encapsulation solution/core mixed suspension is ejected through a nozzle having a single orifice, resulting in a solid spherical bead-like formation.
b) the core solution is ejected through an orifice and the encapsulating solution is ejected through an outer orifice, the resulting encapsulated sphere being then ejected into the cooling solution;
or the use of such a process to make minicapsules in the preparation of a
formulation of any of claims 5 to 11.

13. A method making a seamless minicapsule containing a pharmaceutically active ingredient solubilised or dispersed in a pharmaceutically acceptable liquid phase, and obtainable by a process which utilises surface tension wherein two different solutions which do not dissolve with each other or hardly dissolve with each other contact each other, the process comprising ejecting through a nozzle having a single orifice, and into a cooling solution a shell/core mixed suspension, which outer shell solution is gelled in the cooling solution, wherein the shell part is a gelatin-based solution and the core part is a hydrophobic solution or suspension, the hydrophobic solution or suspension comprising a pharmaceutically active ingredient solubilised or dispersed in a pharmaceutically acceptable liquid phase, the shell of the minicapsule being coated with a coating selected from a polymer coating comprising an ethylcellulose, a small intestinal-specific releasing enteric coating, and a colon-specific releasing coating.

14. The use, in the manufacture of a formulation comprising a plurality of seamless minicapsules, of minicapsules containing an active entity in particle form, the minicapsules being selected from:
a) seamless minicapsules containing an active entity in a solid and/or semi-solid form and an encapsulating medium, the seamless minicapsules having a diameter of from 0.5 mm to 5 mm, the minicapsules being obtainable by dissolving/suspending a powder/solid material in a liquid phase (e.g. gelatin), on cooling/hardening of the liquid phase, the solid material coming out of solution and being present as a solid in the hardened core, optionally wherein the active entity is in the form of nanoparticles;
b) minicapsules formed from a core solution containing an active ingredient, and an encapsulating solution which forms, on setting, an encapsulating medium, the encapsulating solution containing an active ingredient which may be the same as or different from the active ingredient in the core solution and the active ingredient contained in the encapsulating solution being in a micronized or nanonized particle form; or
c) minicapsules formed from a solution containing an encapsulating medium and an active ingredient in a micronized or nanonized particle form.

15. The use of claim 14 wherein the encapsulating solution is a gelatin solution.

16. The subject matter of any of claims 1 to 13 wherein the active ingredient or active entity comprises stem cells or is a live, attenuated or killed mammalian, bacterial or viral cell culture, optionally wherein the cell culture contains a genetically modified organism, e.g. to synthesise and secrete an active molecule, or wherein the active ingredient or active entity is genetically modified mammalian or bacterial cells which are for replacement, for augmentation or for production of therapeutics and which are cultured in a form that maintains viability and activity in the gastrointestinal tract.
